# EUROPEAN PATENT SPECIFICATION

(11) **EP 1 673 048 B1**
(45) Date of publication and mention of the grant of the patent: **19.06.2013**
(21) Application number: 04790607.8
(22) Date of filing: 18.10.2004
(51) Int. Cl.: A61F 2/44, A61B 17/70, A61F 2/30, A61B 17/86

(54) **FLEXIBLE IMPLANT**
FLEXIBLES IMPLANTAT
IMPLANT FLEXIBLE

(30) Priority: 17.10.2003 DE 10348329; 17.10.2003 US 512113 P; 21.11.2003 US 523946 P; 03.03.2004 US 550182 P; 04.05.2004 DE 102004021861
(43) Date of publication of application: 28.06.2006
(73) Proprietor: Biedermann Technologies GmbH & Co. KG, 78166 Donaueschingen (DE)
(72) Inventor: BIEDERMANN, Lutz, 78048 VS-Villingen (DE); MATTHIS, Wilfried, 79367 Weisweil (DE); HARMS, Jürgen, 76227 Karlsruhe (DE)
(74) Representative: Lang, Christian
(86) International application number: PCT/EP2004/011782
(87) International publication number: WO 2005/039454

(56) References cited:
- EP-A- 0 529 275
- EP-A- 0 950 389
- EP-A- 1 273 276
- WO-A-95/19153
- WO-A-99/65425
- WO-A-03/047442
- WO-A-2004/105577
- FR-A- 2 634 371
- FR-A- 2 717 370
- US-A- 5 423 816
- US-A- 5 423 817
- US-A1- 2003 191 470
- US-A1- 2003 220 643
- US-B1- 6 402 750

## Description

The invention refers to an implant for temporary or permanent introduction into a human or animal body.

In modem medicine, many defects in the human or animal body can be compensated or minimized in their effect by the use of implants. For example, space holders for vertebrae or intervertebral discs are known that serve to replace a vertebral body or an intervertebral disc. As a further example, stiffening or stabilization systems for the spinal column may be mentioned in which pedicle screws are fixed in the vertebrae and connected to each other via a connecting rod, such that the distances and arrangement of the vertebrae can be aligned and fixed relative to each other.

With all implants, it is important that materials be used which are compatible with the human or animal organism, that is to say do not cause rejection reactions or lead to a burden on the organism due to disintegration phenomena. Accordingly, the choice of materials for implants is substantially restricted.

In addition, it is advantageous to form the implants as simply as possible and especially from few parts because the composing of the parts necessitates increased outlay for the operator inserting the implants and, on the other hand, through the connection sites of the various parts to each other, there is greater error susceptibility and hence probability of malfunctions. In so far, it is especially preferred to form implants integrally.

As opposed to this, implants must however fulfill different functions, which makes it appear desirable to use different materials and/or to compose implants from several parts. For example, it is desirable for spacers that they all not only fulfill the function of filling the space and holding the vertebrae at a certain distance from each other, but that they also facilitate a certain movement of the vertebrae towards each other, i. e. fill an articulating function within certain narrow limits. For this purpose, it is possible, for example, to provide a space holder in accordance with DE 10056977 C2 in which, between the support elements abutting the vertebral bodies, a bellows-shaped piece of tubing extendable in the longitudinal direction of the implant and made from a tightly woven or knit textile material is arranged. This, however, has the disadvantage described above that several different materials have to be used that must be connected to each other, a fact which can increase the error susceptibility. In addition, there are implants in which a certain flexibility, i. e. especially extensibility and compressibility and bendability, would be advantageous, but this so far has not been taken into consideration on account of the problems described above concerning the connection technique or choice of material.

From EP 0 669 109 B1, a stabilization device for stabilizing adjacent vertebrae is known that comprises two monoaxial pedicle screws and a band which is attached, in the receiving parts of the pedicle screws, respectively by a clamping screw and which said device contains several support elements in the form of a pressure-proof body pulled over the band.

Aside from the disadvantage of a large number of different parts, this stabilization device, however, also has the problem that it is no longer flexible when covered with the support element. The use of monoaxial pedicle screws further limits the use of this stabilization device. A similar stabilization device, in which polyaxial pedicle screws are used instead of monoaxial pedicle screws, is known from EP 1 188 416 A1.

From US 2003/0109880 A1, a dynamic stabilization device for vertebrae is known that comprises a first and a second screw anchored in the vertebrae, each screw having a receiving part for inserting one of the springs connecting the screws and one such spring.

The spring itself is formed as a whole in the shape of a helical spring with densely adjacent threads in the fashion of a tension spring and is fixed via clamping screws in the receiving parts. This, however, has the danger that the spring yields on account of its elasticity to the pressure of the clamping screw and hence the fixing between the bone screw and the spring is loosened.

Further examples for stabilization devices for the spine are found in US 2003/0191470 A1, FR 2 717 and WO 03/47442. Examples for space holders used in spinal applications are found in WO 99/65425 and EP 0 950 389 A2. An example for an hip implant is found in FR 2 634 371. US 5,423,816 shows an intervertebral locking device and US 5,423,817 shows a intervertebral fusing device.

Object of the present invention is therefore to provide implants which are made of the simplest possible parts, especially of one piece or of a few pieces that are easy to connect, and in which, aside from other functions, a certain flexibility and mobility within the implant or regions thereof is to be guaranteed. In addition, these implants are to be easy to manufacture and implant and be safe in operation and to have a long lifetime and diverse application possibilities.

This object is achieved by implants having the features in accordance with claim 1. Beneficial embodiments are the object of the dependent claims.

The invention proceeds from the knowledge that, in the case of implants for human or animal bodies, basically a shape is chosen with which one or more functions that the implant is to effect in the body are fulfilled. These functions can also include the implant's providing a certain degree of mobility or elasticity. In other cases, again, elasticity or mobility within the implant is basically not necessary, but possibly desirable and advantageous. In this regard, in the following, the mobility and elasticity function will be designated the second function while all other functions represent first functions.

In the first group of cases, the prior art is such that the mobility or elasticity is obtained through additional parts and/or different types of materials. The invention takes another route in which the flexibility or mobility is not effected by another material or by the provision of additionally separate parts, but rather, in the case of an integral implant or implant part, the area which is to have the flexibility or mobility is achieved by making provision in the design for material recesses.

In the second group of cases, in which flexibility or mobility is not an absolutely necessary function, this additional function is also effected, in accordance with the invention, by the provision of corresponding material recesses in the corresponding areas, with this occurring in addition to the shape specified by the functions to be obtained.

In this way, by eschewing additional elastic materials and corresponding connecting parts or additional separate parts, flexibility and mobility can be obtained simply within the implant or parts thereof. In this regard, the elasticity or mobility functions can be provided in addition to the necessary functions of the implant or as a component of the necessary functions.

Especially, it is possible in this way to realize compression and/or expansion zones and, within certain limits, bending joints or torsional elements and the like in a simple and reliable manner, especially in an integral implant or implant part.

Correspondingly, it is possible to use for the preferably integrally formed implant a stable, stiff, especially for the intended conditions of use, a rigid, preferably flexible rigid material, such as titanium, titanium alloys, plastics and the like. Generally, all biocompatible materials are candidates that do not cause rejection reactions or show any disintegration phenomena that are a burden on the body.

The material recesses can preferably be provided in the form of groove-shaped recesses or open apertures of walls of the implant or implant part. The shape, number and arrangement of the material recesses can be adjusted from case to case to the load requirements.

As a universal shape that satisfies diverse requirements, the material recess can especially be provided in a helix shape running around the implant body, such that especially the shape of a type of helical spring results, with its being especially advantageous in this case that free spaces are present between adjacent fillets of the helical spring element on account of the material recess. Aside from easier manufacturability and the associated larger choice of material, this has the advantage of achieving greater flexibility.

Especially advantageously, two material recesses can be provided that are formed as twin-track or two-flight helixes. In this way, two helical springs arranged inside each other can especially be formed. If the area of the helix-shaped recess has the same height, two helix-shaped recesses of double pitch can be provided instead of one helix-shaped recess of a low pitch.

Especially suitable for the correspondingly flexible implant are space holders for vertebrae and/or intervertebral discs and connecting rods of pedicle screw arrangements, which are advantageously used together as a system according to the present invention, to enable the patient with a correspondingly stabilized spinal column to have adequate mobility.
The space holders for vertebrae and/or intervertebral discs provide a space-holder and weight-transfer function as first functions, whereas damping effect and mobility come additionally as second function.

As regards connecting rods, supporting and connecting functions are to be mentioned as first functions.

For the space holders or connecting rods, it has proved advantageous to form these as a tube-like body with a central tube-like body part as well as connection elements provided on the ends, with the material recesses responsible for the flexibility provided preferably in the form of one or two helix-shaped apertures in the tube-like body part, such that this part essentially has the shape of one or two helical springs arranged inside each other. The connection elements of the space holders preferably have corresponding means of connecting the space holder with adjacent body parts, such as vertebrae, in the form of hook-like projections on the ends and/or recesses, grooves and openings on the jacket surface so that the space holder may grow into and knit with the tissue. In this regard, however, the cavities or recesses of the connection elements must not be confused with the material recesses for attaining flexibility and mobility of the space holder in the tube-like body part. Since the connection elements knit completely with the adjacent body parts, such as the vertebrae, they do not contribute to the flexibility or mobility of the vertebrae.

The means for connecting the tube-like body to adjacent body parts may be arranged either integrally with the tube-like body especially in extension of this body on the ends or detachably on the ends, such as on end plates that can be screwed onto the ends of the tube-like body.

Such detachable end plates or end plates connected integrally with the tube-like body are preferably provided when, around the tube-like body with the material recesses, at least one sleeve of elastic material is arranged for the purpose of achieving elasticity or mobility, or, within the tube-like body, at least one elastic core is provided. Such an elastic core or elastic sleeve of preferably one elastomer offers the advantage that it allows the elasticity or rigidity of the tube-like body or space holder to be precisely adjusted. Through the modular-like arrangement of tube-like body with corresponding recesses and core and/or sleeve, the use of different components of different rigidity can effect in a simple manner an exactly defined rigidity of the implant in the sense of damping. In so far, a combination of an implant part with material recesses for achieving flexibility and an implant part consisting of one flexible material for adjusting a defined rigidity is quite generally an object of the current invention.

To achieve an altered rigidity, only the composition of the components has to be changed, i. e. , for example, a different core of different rigidity or a different sleeve need be used with the flexible tube-like body. Although it is conceivable that a sleeve and a core can be used simultaneously together with a flexible tube-like body, for the sake of simplicity it will usually only be a combination of tube-like body and core or tube-like body and sleeve. In this regard, the sleeve also offers the advantage of protecting the tube-like body with the preferably helix-shaped recesses against external influences, whereas, as opposed to this, when a core is used, the core is protected by the tube-like body.

Both core and sleeve can advantageously be held by the arrangement of end plates on the ends of the tube-like body, with, in the case of the arrangement of a sleeve, the end plates projecting preferably beyond the tube-like body and thus having a larger diameter than the tube-like body. The end plates can at least partly, that is on one side, be connected integrally with the tube-like body, such that a beaker-like shape is obtained here. In addition, the end plates can be connected detachably either on one side or on two sides to the tube-like body, for example via a screw or thread connection. In this regard, the outer thread can be provided both on the end plate and on the tube-like body.

Preferably, the implant or the tube-like body with the material recesses for obtaining flexibility and mobility is extensible or compressible in its longitudinal direction along the space holder longitudinal axis by 0.5 to 20%, especially 1 to 15%, and bendable about a radial axis perpendicular to the longitudinal axis of the space holder, such that adjacent body parts can be pivoted by approximately 0.5 to 10, especially 1 to 6 degrees with reference to the longitudinal axis. In addition, in a preferred embodiment, a torsional movement of 0.5 to 2. 5 about the longitudinal axis is possible.

In a further advantageous application of the flexible implant, a connecting rod between monoaxial or polyaxial pedicle screw arrangements in the region between the pedicle screws arrangements can, by arrangement of corresponding material recesses, receive a certain flexibility and mobility that can especially be enforced by the at least partial formation of the rod as a hollow body. The rod-shaped element is especially suited for use in the stabilization and mobility limitation of contiguous vertebrae in the case of intervertebral disc defects of different severity. These properties are to be realized during manufacture in a simple manner by changing the dimensions of the rod-shaped element.

Such an implant can, for example, be manufactured in a simple manner from a body, such that one or more material recesses can be incorporated into the wall of the body by mechanical or chemical milling, EDM, laser treatment or any other way. Especially, one or preferably two material recesses can be provided around the body in a helix shape or along the wall.

In as far as the body is a solid body, such as a solid cylinder, it is possible, in a preceding or subsequent second step, to make a bore hole, especially coaxial to the material recess or material recesses, such that a helical spring shape is preferably formed in the elastic region.

When pipe material is used, drilling of the core can be dispensed with.

Preferably, the material recess can be made by laser treatment of a pipe-like body because in that case two recesses can be incorporated in one processing step. By means of laser, it is namely possible to incorporate bore holes simultaneously on two sides by boring right through the pipe-like body (for example, quadratic pipe as well). Turning and advancing the body simultaneously generates a double-flight helix.

In a further aspect, the invention refers to a rod-shaped element for connecting at least two bone-anchoring elements, which each have an anchoring section for anchoring in the bone and a receiving part for connecting with the rod-shaped element, with at least one rigid section and one elastic section, which are formed from one piece, with especially the elastic section being formed as a helical spring, and/or, in which at the opposite end of the elastic section abutting the rigid section, a second rigid section is provided adjacent to the elastic section, and/or in which the outer diameter of the elastic section is different in at least one position from the outer diameter of the rigid section, and/or in which the elastic section in a certain direction perpendicular to the rod axis has a smaller outer diameter at least in some part than in another direction, and/or in which the outer diameter of the elastic section varies, and/or in which the elastic section has a core, and/or in which a coaxial bore hole stretching through the rod-shaped element is provided.

Accordingly, the invention refers to a stabilization device for spinal application with at least two bone-anchoring elements, each having a bone-anchoring section for anchoring in the bone and a receiving part, and a rod-shaped element, as described above, for connecting to the bone-anchoring elements, with especially the bone-anchoring element being a monoaxial or a polyaxial bone screw.

Following is an example of a process for making a rod-shaped element comprising the steps:
a) provision of a rigid rod;
b) generation of a helical spring section on at least one longitudinal section of the rod in a predetermined distance from the free end of the rod, preferably by a material-removing method,
   with, especially, a core being drilled in an axial direction or left, and/or a defined material area is removed in the longitudinal direction of the elastic section for generating a noncircular cross-section in at least one area of the elastic section, and/or the diameter of the rigid section is reduced in relation to the elastic section.

The present invention particularly refers to an elastic element for use in a connecting rod of the implant for spinal application or vertebrae, provided as an essentially cylindrical body with a first end and a second end opposite thereto, with the opposite ends of said body comprising a coaxial hole each and at least one of these ends comprising an internal thread for connecting to a shaft and/or head of a bone screw or for connecting to a rod section or plate, wherein particularly an internal thread is provided at each of the two ends.

The present invention further refers to an elastic element for use in a connecting rod of the implant for spinal application and vertebrae, provided as an essentially cylindrical body with a first end and a second end opposite thereto, with the first end of said body comprising a cylindrical projection with an external thread for connecting to a shaft or to a head of a bone screw, for connecting to a rod section or for connecting to a plate.

The second end of said body advantageously comprises a cylindrical projection with an external thread for connecting to a shaft or to a head of a bone screw, for connecting to a rod section or for connecting to a plate.

According to a further preferred embodiment, the elastic element comprises a coaxial bore hole adjacent to its second end and/or at least in a section of the coaxial bore hole that is adjacent to the second end, an internal thread for connecting to a shaft or to a head of a bone screw, for connecting to a rod section or for connecting to a plate.

According to a further preferred embodiment, the elastic element is characterized in that the bore hole extends over the entire length and/or in that the body is provided to be tubular in shape with a continuous coaxial bore hole and a recess in the wall that extends in the form of a helix in the direction of the cylinder axis, wherein, in radial direction, the recess ends in the bore hole.

According to still another preferred embodiment the elastic element is characterized in that a core is provided in the bore hole and/or in that the elastic element is provided as a helical spring.

According to still another preferred embodiment, the elastic element is made from a body-compatible material, in particular titanium.

The present invention particularly refers to a bone anchoring element with an elastic element as described above, comprising a shaft with a bone thread that is connected to the one end of the elastic element, and an end piece, preferably a head, of a bone screw that is connected to the other end of the elastic element.

The present invention particularly refers to a rod-shaped element for connecting two bone anchoring elements with an elastic element and a first rigid rod section that is connected to the one end of the elastic element, wherein particularly a second rigid-rod section is connected to the other end of the elastic element.

The present invention particularly refers to a plate with a cylindrical projection with an external thread or with a bore hole with an internal thread at at least one end of the plate, for connecting to a flexible or elastic element as described above.

The present invention particularly refers to a connecting rod of the implant for the dynamic stabilization of the spinal column with at least two bone anchoring elements that are connected to each other by means of a rod-shaped element as described above.

Following is an example of a method for the manufacture of an elastic element comprising the following steps:
(a) providing a tube-like body or
(b) providing a body that is cylindrical in shape
(c) forming a helix-shaped recess by removing material, form outside, by metal-cutting along a helix that extends coaxial to the main axis of the cylindrical or tube-like body;
(d) forming a bore hole along the main axis of the cylindrical body;
(e) forming an internal thread in one of the two end sections of the bore hole or the tube-like body;
wherein in particular the internal diameter of the bore hole of step d) is selected such that the helix-shaped recess in the outside wall of the cylindrical body formed by metal-cutting in step c) ends in the bore hole in radial direction,
wherein in particular an internal thread in the other end section of the bore hole is formed.

Following is another example of a method for the manufacture of an elastic element comprising the following steps:
(a) providing a cylindrical body;
(b) forming one cylindrical projection with an external thread on each of the two ends of the cylindrical body by means of metal-cutting turning;
(c) forming a helix-shaped recess by removing material, from outside, by metal-cutting along a helix that extends coaxial to the main axis of the cylindrical body;
(d) forming a bore hole along the main axis of the cylindrical body advantageously further comprising the following steps:
(f) finishing by means of milling, the runout of the helix-shaped recess after forming the bore hole in order to remove a sharp edge on the inside of the bore hole; and
(g) deburring the elastic element thus formed.

Following is yet another example of a method for the manufacture of an elastic element comprising the following steps.
(a) providing a tube-like body with a first and a second end
(b) providing a cylindrical body with a first and a second end and forming a bore hole coaxial to the main axis of the cylindrical body with said bore hole being adjacent at least to the first end of the cylindrical body;
(c) cutting by means of wire-EDM, laser treatment or water jet treatment of a recess along a helix extending coaxial to the main axis of the cylindrical body;
(d) either forming, by means of metal-cutting turning, a cylindrical projection with a diameter that is smaller than the predetermined external diameter of the cylindrical or tubelike body provided in steps (a) or (b) and forming an external thread on the surface of the cylindrical projection at the first end of the cylindrical body, or forming an internal thread in the bore hole in a section adjacent to the first end of the cylindrical body or the tube-like body, wherein particularly the bore hole formed in step (b) extends from the first to the second end of the cylindrical body.

Furthermore a method can comprise the steps of forming an internal thread in the bore hole in a section adjacent to the second end of the cylindrical body.

A method can further comprises the step of forming, by means of metal-cutting turning, a second cylindrical projection with a diameter that is smaller than the predetermined external diameter of the cylindrical or tube-like body, and forming an external thread on the surface of the second cylindrical projection at the second end of the cylindrical or tube-like body.

Following is a further example of a method for the manufacture of an elastic element comprising the following steps:
(a) providing a cylindrical or tube-like body with a first and a second end;
(b) forming, by means of metal-cutting turning, a cylindrical projection with a diameter that is smaller than the predetermined external diameter of the cylindrical or tube-like body provided in step (a), and forming an external thread on the surface of the cylindrical projection at the first and at the second end of the cylindrical body; and
(c) cutting, by means of wire-EDM, laser treatment or water jet treatment, of a recess along a helix extending coaxial to the main axis of the cylindrical body, wherein particularly the two runouts of the helix-shaped recess are provided in the form of a quarter circle.

Further advantages, characteristics and features of the present invention become apparent from the following detailed description of two embodiments with the aid of the enclosed drawings. The drawings show in a purely schematic manner
- Fig. 1: a three-dimensional view of a space holder for vertebrae or intervertebral discs;
- Fig. 2: a lateral view of the space holder from Fig. 1;
- Fig. 3: a detailed lateral view of the space holder from Figs. 1 and 2;
- Fig. 4a) -c): views of a further space holder;
- Fig. 5a) -c): views of a third space holder;
- Fig. 6a) -c): examples for stages of the production of a body;
- Fig. 7: a three-dimensional view of two pedicle screw arrangement with a connecting rod and a space holder;
- Fig. 8: a three-dimensional view of two adjacent vertebrae with space holders arranged in between and a lateral fixing by pedicle screw arrangements with flexible connecting rod
- Fig. 9: a schematic three-dimensional view of the stabilization device with a rod-shaped element in accordance with the invention;
- Fig. 10: a three-dimensional representation of the rod-shaped element according to Fig. 9;
- Fig. 11a): a lateral view of the rod-shaped element according to Fig. 9;
- Fig. 11b): a sectional view of the rod-shaped element according to Fig. 9;
- Fig. 12a): a three-dimensional view of the connection between a rod-shaped element and bone anchoring elements;
- Fig. 12b): a sectional view of the connection between a rod-shaped element and bone anchoring elements;
- Fig. 13: a lateral view of a rod-shaped element according to a further embodiment;
- Fig. 14: a lateral view of a rod-shaped element according to a further embodiment;
- Fig. 15: a lateral view of the rod-shaped element of Fig. 14 turned through 90 ;
- Fig. 16: a three-dimensional view of a rod-shaped element according to a further embodiment;
- Fig. 17: a lateral view of the rod-shaped element according to Fig. 16;
- Fig. 18: a sectional view of a rod-shaped element according to a further embodiment;
- Fig. 19: the operation of a stabilization device of the invention with a rod-shaped element;
- Fig. 20: a lateral view of the rod-shaped element according to Fig. 19;
- Fig. 21: a three-dimensional view of a stabilization device with rod-shaped element in accordance with Fig. 19 in a second state;
- Fig. 22: a lateral view of the rod-shaped element of Fig. 21;
- Fig. 23: a further application of the stabilization device;
- Fig. 24: a further application example of the stabilization device;
- Fig. 25a: a lateral view of a further embodiment of a flexible rod-shaped element;
- Fig. 25b: a sectional view of the flexible rod-shaped element of Fig. 25a;
- Fig. 26a: a first application example of the flexible rod-shaped element;
- Fig. 26b: a modification of Fig. 26a;
- Fig. 27: a bone anchoring element with the flexible rod-shaped element of Fig. 25a and b;
- Fig. 28: a stabilization device, consisting of two three-piece bone anchoring elements and one rod-shaped element, each comprising a flexible element;
- Fig. 29: a lateral view of a further embodiment of a flexible rod-shaped element;
- Fig. 30a: a lateral view of still a further embodiment of a flexible rod-shaped element;
- Fig. 31a: a lateral view of a flexible rod-shaped element according to another embodiment;
- Fig. 31b: a lateral view, turned by 90 degrees, of the flexible rod-shaped element of Fig. 31a;
- Fig. 32: a sectional view of a flexible rod-shaped element;
- Fig. 33: a flexible rod-shaped element according to a further embodiment of the invention;
- Fig. 34: a flexible rod-shaped element according to a further embodiment of the invention;
- Fig. 35a: an exploded view of a joining element consisting of a rod-shaped element, a flexible element according to the invention, and a plate;
- Fig. 35b: a sectional view of the plate of Fig. 35a along the line A-A;
- Fig. 36: an application example of the plate of Figs. 35a and 35b, in which the plate and the rod-shaped element connected to the plate by means of a flexible element are each anchored in vertebrae by means of bone anchoring elements;
- Fig. 37a: is an example not part of protected matter of an application of a variation of the flexible rod-shaped element in a dynamic stabilization device for a pelvic bone;
- Fig. 37b: a sectional view of a bone anchoring element used in the stabilization device of Fig. 37a;
- Fig. 38: a spring element manufactured by means of wire-cut electrical discharge machining (wire-cut EDM), laser treatment or water jet treatment.

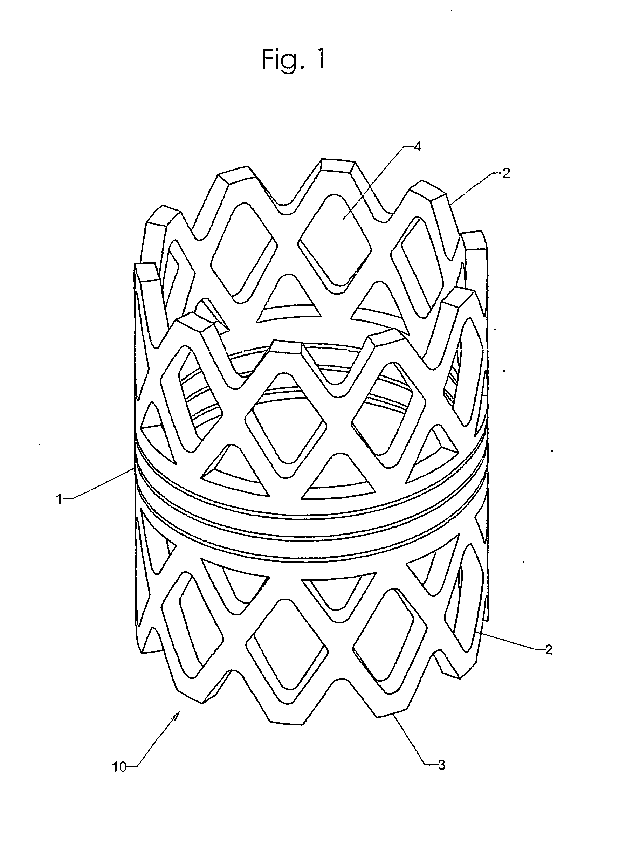

Fig. 1 is a three-dimensional view of a first embodiment of the space holder of an implant, in accordance with the invention, in the form of a space holder for vertebrae or intervertebral discs. Space holder 10 has a cylindrical body 1 and two connection elements 2 provided at the ends of the cylindrical body 1 for connecting the space holder 10 to the adjacent body parts, e. g. bones or cartilage in, for example, the human body.

Connecting elements 2, which are arranged at the ends of cylinder-shaped body 1, have identical shapes in the sample embodiment shown, but may also have different shapes.

Connection elements 2 have serrations 3 on each free end of their ends that can engage with the adjacent body tissue at the site of implantation.

Serrations 3 are formed by triangular recesses 5 on both ends of space holder 10, such that trapezoidal serrations 3 are formed that can engage with and cling on to adjacent body tissue.

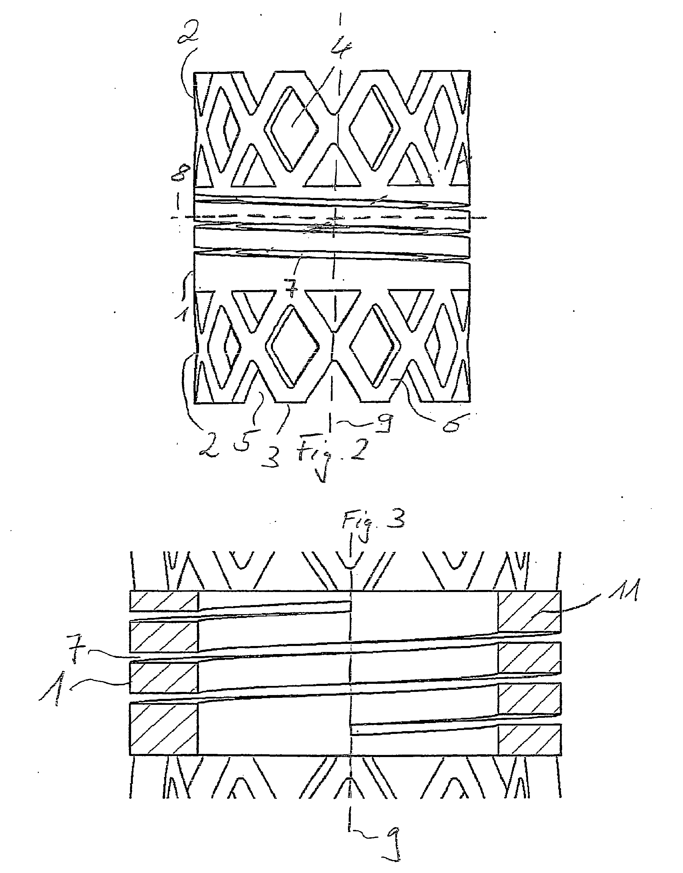

In addition, connection elements 2 have diamond-shaped cavities 4 (see Fig. 2), so provided that they are adjacent to each other around the entire cylinder jacket surface of connection elements 2. As a result, the respective connection element is formed again to itself by a large number of diamond-shaped interconnected fillets 6, with the tips of the diamonds formed by fillets 6 cut-off so that trapezoidal sensations 3 are formed.

Tube-like body 1 between connection elements 2 on each end of the cylinder has, in the embodiment shown, a helix-shaped material recess 7, such that the wall 11 (see Fig. 3) itself assumes a helix shape. Since space holder 10 is otherwise formed overall as a hollow cylinder, tube-like body 1 between connection elements 2 with material recess 7 represents an elastic area or a movement area, even if space holder 10 itself is formed from an essentially stiff material, such as titanium or a titanium alloy. Through material recess 7, space holder 10 receives a design-related elasticity in the region of the tube-like body 1, which makes it possible to dispense with provision of a separate elastic material in this area for obtaining a separate elasticity or mobility. Especially, this can avoid having to produce the space holder from several parts that have to be fitted together.

Through helix-shaped material recess 7 is obtained in a simple manner an extensibility and compressibility of tube-like body 1 along longitudinal axis 9 of space holder 10 and a bendability about a rotary axis perpendicular to longitudinal axis 9, which for example is illustrated by axis 8 (Fig. 2). Here, especially the helix shape of material recess 7 has proved its worth, which facilitates a balanced elasticity or mobility in the most diverse directions.

Naturally, however, other shapes of material recesses and a different number and arrangement of these material recess are possible and conceivable, with solutions adapted to individual cases or the load profile being possible.

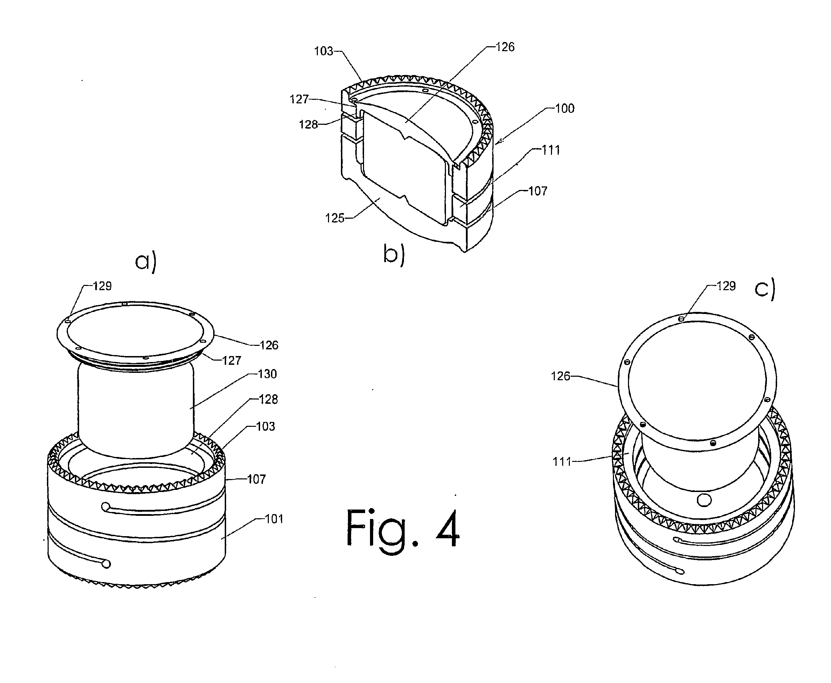

Fig. 4 shows in sub-figures a) to c) three different exploded three-dimensional views and sectional views (b) of a second embodiment of a space holder 100 with a tube-like body 101, which is sealed on the lower side by an end plate 125 connected integrally with the tube-like body 101, such that a beaker-like shape results.

Tube-like body 101 has in its walls 111 a helix-shaped recess 107 that imparts a certain flexibility in accordance with the invention to the tube-like body 101.

To be able to exactly adjust the stiffness of space holder 100, inside tube-like body 101 is provided a swappable core element 130 of an elastomeric material, which is held on the lower side by end plate 125 and on the upper side by end plate 126 in tube-like body 101.

End plate 126 on the upper side of the space holder has an external thread 127, by means of which it can be screwed into internal thread 128 of tube-like body 101 on the upper end in the inside of tube-like body 101, End plate 126 has a shoulder with which it lies tightly against wall 111. Serrations 103 are provided all around the end of wall 111 and project over end plates 125 and 126 and can engage with adjacent tissue in order to hold the space holder firmly in position there.

End plate 126 has engagement openings 129 by means of which end plate 126 can be screwed into the tube-like body 101.

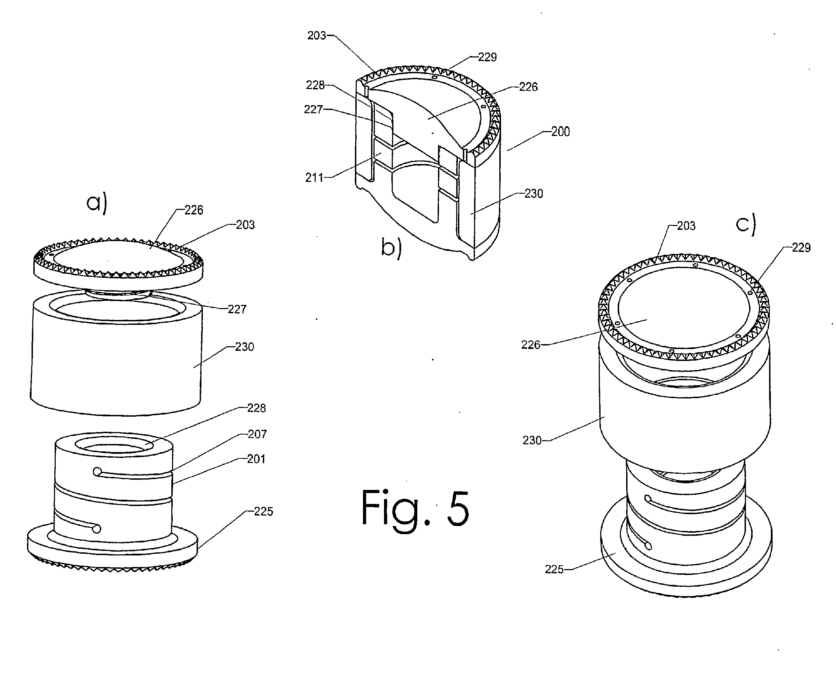

Fig. 5 shows in sub-figures a) to c) a third embodiment of a space holder, with sub-figures a) to c) representing exploded three-dimensional diagrams, while sub-figure b) shows a three-dimensional sectional view. In the embodiment of Fig. 5, tube-like body 201 again has a helix-shaped recess 207 in body wall 211.

On the lower side, tube-like body 201 is again closed by an integrally arranged end plate 225, such that here again a beaker-like shape of tube-like body 201 results. Admittedly, end plate 225 is formed such that it has a larger outer diameter than tube-like body 201, in which the helix-shaped material recess 207 is arranged. Thereby is created a shoulder that forms a receptacle for a tube-like sleeve 230 of elastomeric material. Elastic sleeve 230 is pushed over tube-like body 201 such that this is completely surrounded by the sleeve. On the upper end, an end plate 226 is screwed on to tube-like body 201 by means of a thread connection.

In this regard, the outer thread 227 of end plate 226 engages with internal thread 228 of tube-like body 201, such that sleeve 230 is held firm between end plates 225 and 226.

Sleeve 230 also serves to adjust the overall rigidity, in that simply exchanging sleeve 230, in a manner similar to exchanging core 130 (see Fig. 4), makes it possible to simply vary the rigidity of the overall implant 100 and 200.

Pyramid-shaped serrations 203, which serve to engage with the adjacent tissue so as to firmly anchor the space holder, are provided on end plates 226 and 225.

Lid 226 also has engagement openings 229, by means of which end plate 226 can be screwed onto tube-like body 201.

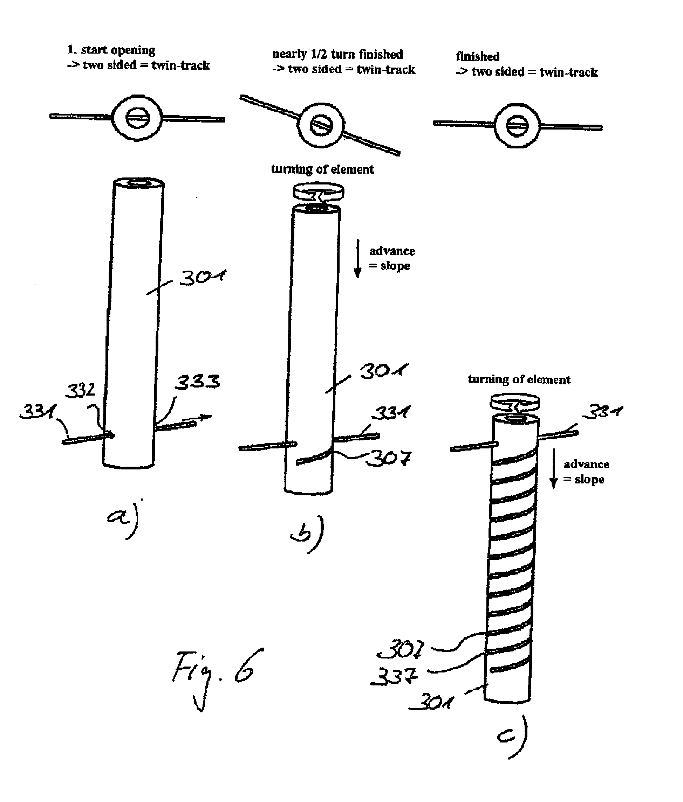

Fig. 6 shows an example of how, by means of laser treatment, a laser beam 331 can simply introduce two material recesses 307 and 337 into pipe 301.

As may be seen in sub-figure a) of Fig. 6, pipe 301, as especially evident also from the plan view, is drilled through first with laser beam 331, such that two openings 332 and 333 result. Then, by rotating pipe 301 and simultaneously advancing along the arrow, as indicated in Fig. 6 b), starting from opening 332, material recess 307 and, starting from opening 333, material recess 337 are incorporated. As a result, two parallel material recesses are generated, such that twin-track, two-pitch helical springs arranged inside each other or helical spring areas result.

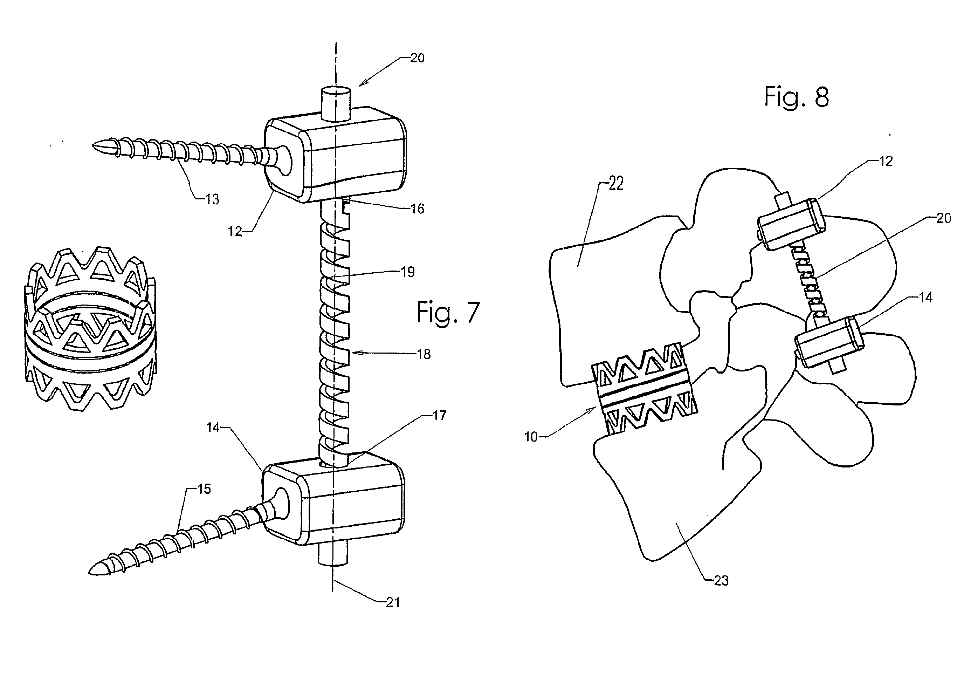

An embodiment of a pedicle screw arrangement of the flexible implant of the invention is shown in Fig. 7. Fig. 7 shows in a three-dimensional view two pedicle screw arrangements 12 and 14 with the respective pedicle screws 13 and 15, which are connected to each other by a connecting rod 20.

Connecting rod 20 is designed as a flexible implant, and more precisely in elasticity or movement area 18, which is arranged between holding areas 16 and 17, in which the connecting rod is held by the pedicle screw arrangements.

A helix-shaped material recess 19 is provided in flexible area 18 of connecting rod 20 and extends around the longitudinal axis 21 of connecting rod 20.

Since connecting rod 20 is designed as a hollow cylinder, the elastic area 18 also has an essentially helix shape.

For producing the connecting rod of the invention shown in Fig. 7, a solid cylinder or rod of a biocompatible material, such as a titanium alloy, may be used. Into this solid rod is incorporated in a first step material recess 19 by mechanical or chemical milling or by laser treatment in the desired area, in other words in flexible area 18. A connecting rod of this kind would already have increased elasticity in the flexible area 18 and could be used as such.

The elasticity or movement possibility in flexible area 18 can be further enhanced by incorporating, also mechanically or chemically, a bore hole along longitudinal axis 21 of the connecting rod, such that rod 20 is given a hollow-cylinder shape or pipe shape.

If the diameter of the bore hole is chosen such that the remaining wall of the connecting rod is smaller than the depth of previously incorporated material recess 19, then, instead of a groove-like recess, an open aperture is present in flexible area 18 and this essentially receives a helix shape as well. Although this last mentioned shape is the preferred embodiment, the preceding steps with merely material recesses provided, that is to say, groove-like recesses or additional formation as hollow body, are possible alternatives.

Fig. 8 shows an application of the implant according to the invention, namely the arrangement of space holder 10 between two vertebrae 22 and 23 and the provision of flexible connecting rod 20 between two pedicle screw arrangements 12 and 14 on the spinal column of a human body. As may be readily seen from this figure, the flexible formation of the implant parts results in a certain mobility of the spinal column in the corresponding areas, a fact which leads to a substantial increase in comfort for the patient, especially in a combined application.

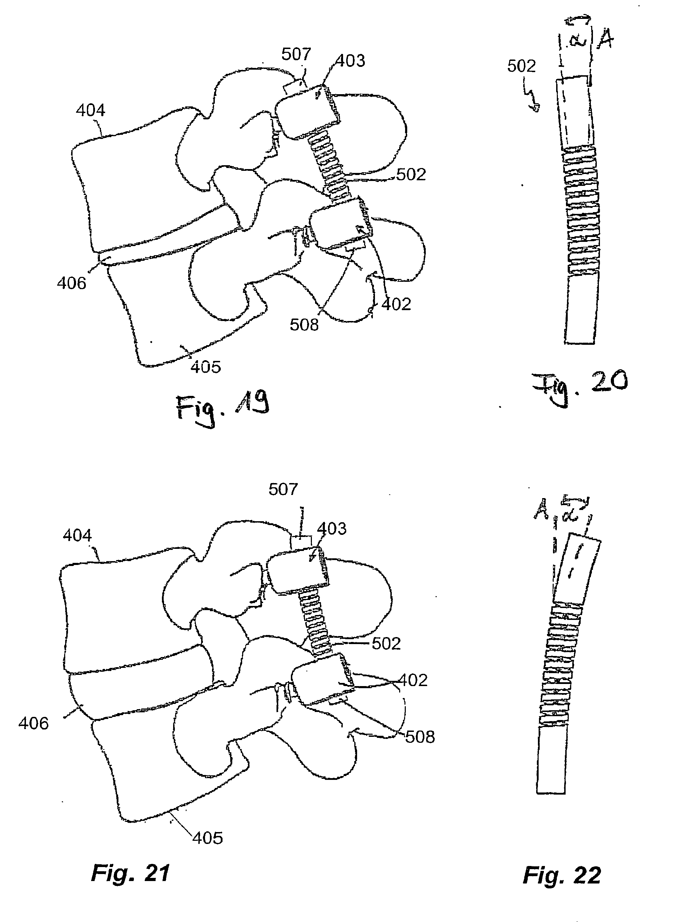

Figures 19 to 36 show further embodiments and application areas of a flexible implant or the corresponding connection rod and space holder, especially how a connecting rod is used in stabilization of the spinal column.

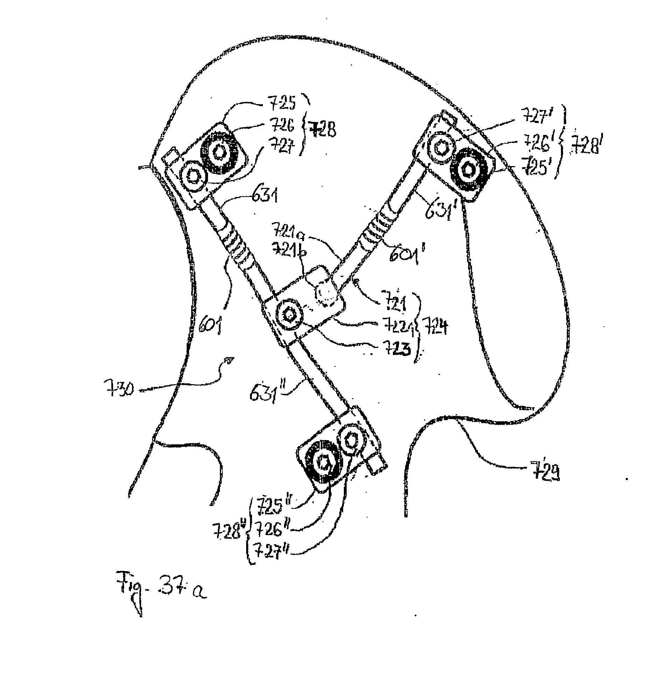

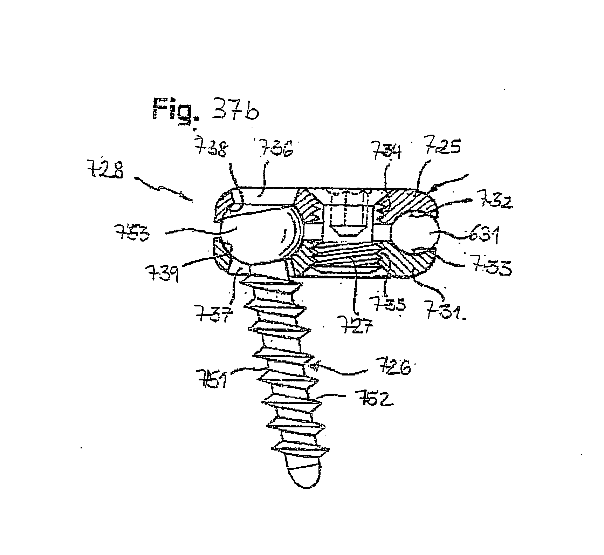

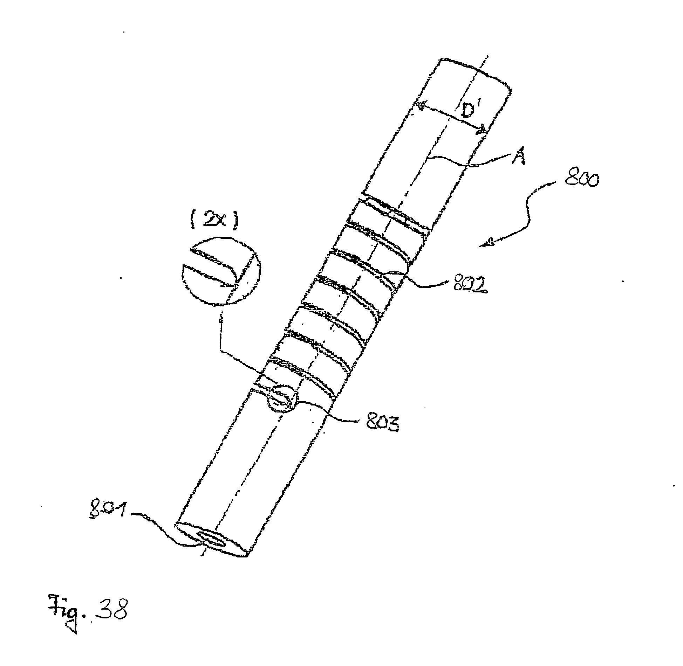

Figures 37 and 38 show a variation of the pedicle screw arrangement not within the scope of the protected matter as an example how variations could be used in non-spinal applications.

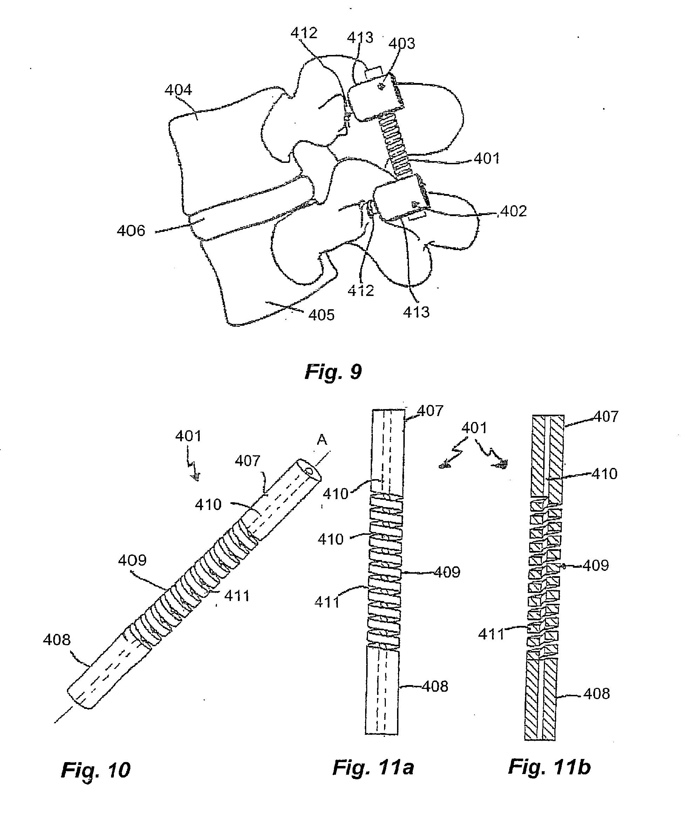

As may be seen from Fig. 9, the stabilization device in the application shown comprises a rod-shaped element 401 and two pedicle screws 402,403 which are connected to each other by the rod-shaped element. Pedicle screws 402,403 are anchored in the pedicles of two contiguous vertebrae 404,405, between which a damaged intervertebral disc 406 is located.

The rod-shaped element 401 of the invention is formed integrally. According to a first embodiment, as shown in Figs. 10, 11 a and 11b, it has a first rigid section 407 stretching from its first end for a predetermined length and a second rigid section 408 stretching from its second end for a predetermined length and an elastic section 409 of predetermined length stretching between rigid sections 407,408, with all sections having the same outer diameter.

Through the rod-shaped element moreover extends a coaxial bore hole 410 of predetermined diameter. Elastic section 409 is formed as a helical spring with threads 411 of a predetermined pitch. The height of threads 411 of elastic section 409 in the direction of longitudinal axis A of the rod-shaped element, the diameter of coaxial bore hole 410, which determines the thickness of threads 411 in radial direction, and the pitch are chosen such that a desired rigidity is obtainable towards axial forces, bending forces and torsional forces that act on rod-shaped element 411.

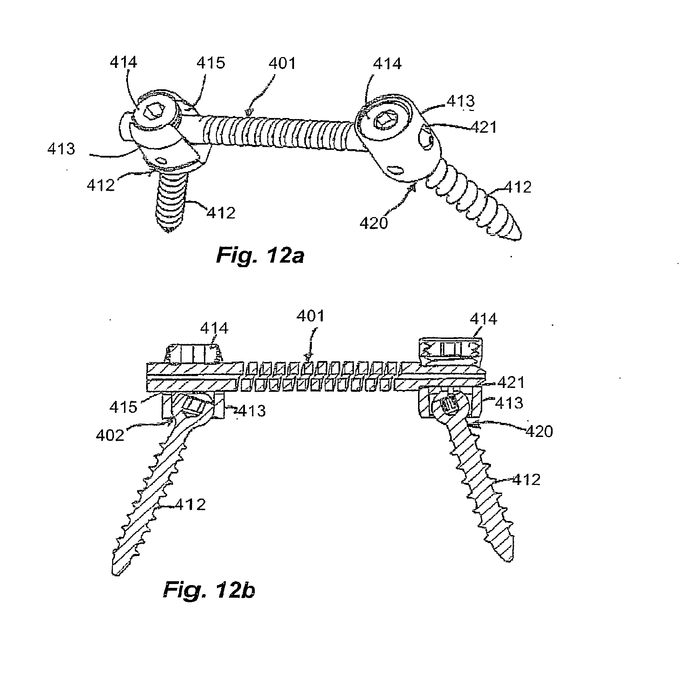

As may be seen from Fig. 9, Fig. 12a and Fig. 12b, pedicle screws 402,403 of the stabilization device have in known manner a thread shaft 412 with a bone thread and an essentially cylindrical receptacle 413 with a U-shaped recess 415 for inserting the rod-shaped element.

To fix the rigid sections 407,408 in receptacle 413, internal screws 414 are provided in the known manner that can be screwed into the receptacle. The pedicle screws are preferably formed as polyaxial screws. The axial length and the diameter of rigid sections 407,408 of rod-shaped element 401 are dimensioned such that rod-shaped element 401 with its rigid sections 407,408 may be joined to pedicle screws 402,403. The length of rigid sections 407,408 therefore corresponds at least to roughly the diameter of internal screw 414, which is provided for fixing the rod-shaped element. In the case of receptacle 413' of pedicle screw 420, into which the rod-shaped element is not inserted from above, but rather pushed laterally into an opening 421, the length of the rigid section also corresponds to at least roughly the diameter of fixing element 414 that fixes the rod-shaped element in receptacle 413'.

In the example of the stabilization device shown in Fig. 9, the length of elastic section 409 of rod-shaped element 401 is chosen such that it essentially corresponds to the distance between pedicle screws 402,403 in the unloaded state of intervertebral disc 406. Elastic section 409, however, can also be longer or shorter.

Rod-shaped element 401 is made of a biocompatible material, such as titanium or a biocompatible plastic, that, however, has little or no elastomer properties.

In operation, first pedicle screws 402,403, 420 are screwed into the vertebrae contiguous with the pedicles and then rod-shaped element 401 with its rigid sections 407,408 are each inserted into one of the receivers 413,413' of pedicle screws 402,403, 420. After positioning of vertebrae 404,405 relative to each other and adjustment of pedicle screws 402,403, 420 relative to the rod-shaped element, rigid sections 407,408 are fixed in receptacle 413,413'. Positioning of vertebrae 404,405 relative to each other in an application proceeds such that elastic section 409 of rod-shaped element 401 is in the resting position in the unloaded state of intervertebral disc 406. When load is applied, forces act on the intervertebral disc 406 via the vertebrae and the intervertebral disc apparatus.

Rod-shaped element 401 limits, through elastic section 409, multiaxial movement of the vertebrae relative to each other and so prevents excessively large forces from acting on the intervertebral disc. Thus, the degeneration process of a slightly or moderately defective intervertebral disc can be stopped. Alternatively, depending on the indication, a predetermined distraction of the vertebrae is carried out already in the unloaded state of the spinal column via the stabilization device, to relieve the intervertebral disc in this way.

Alternatively, again depending on the indication, bone screws can be anchored laterally in the vertebral bodies direct.

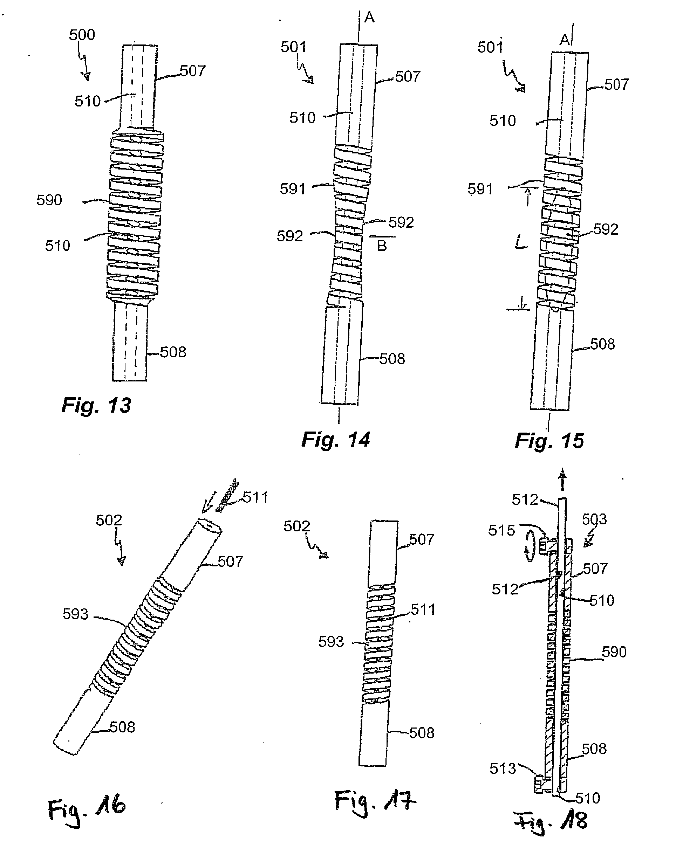

In the embodiment shown in Fig. 13, a rod-shaped element 500, as in the preceding embodiment, has rigid sections 507,508 and an elastic section 590 in the form of a helical spring connected integrally to these between rigid sections 507,508. This differs from the first embodiment in that the diameter of elastic section 590 is greater than the diameter of rigid sections 507,508. Consequently, greater rigidity is obtained relative to the rigidity of the rod-shaped element in accordance with the first embodiment. Operation is as given under the first embodiment.

Figs. 14 and 15 show in a further embodiment a rod-shaped element 501. This differs from rod-shaped elements 401,500 of the preceding embodiments in that elastic section 591 provided between rigid sections 507,508 has two areas 592 offset at 180 to each other and shaped concave to the rod axis. The length L of areas 592 in the direction of the rod axis is at most equal to the length of elastic section 592 and the radius of curvature is such that the threads of the helical spring are not interrupted. As a result of this formation, elastic section 591 is waisted in a direction B perpendicular to rod axis A and thus has a lower rigidity in this direction. As a result, oriented rigidity is obtained, which is expedient for certain applications.

Operation proceeds as under the preceding embodiments with the sole difference being that rod-shaped element 501 can be attached in an oriented manner in the pedicle screws in the circumferential direction. By choosing the dimensions of the spring section, a desired rigidity can be precisely chosen and set

In a further embodiment shown in Figs. 16 and 17, rod-shaped element 502 has a cylindrical core 511 extending coaxially through elastic section 593, said core having a certain flexural elasticity. The diameter of core 511 is dimensioned such that the core is held snugly after being pushed into bore hole 510. The core is preferably made out of the same material as the rod-shaped element, but it can also consist of a flexible plastic.

In a variant, core 511 is connected integrally with rigid sections 507,508 and with the threads of the helical springs of elastic section 593.

Core 511 effects greater flexural rigidity of rod-shaped element 502 compared with the preceding embodiments. Thus, in this embodiment, rigidity can be obtained similar to that of rod-shaped element 500, which has the greater diameter in the elastic section. The flexural rigidity is furthermore adjustable by the choice of the diameter and/or the material of the core.

Operation proceeds as under the preceding embodiments. Unlike the preceding embodiments, however, compression or extension of elastic section 593 in axial direction and torsion are dimensionally reduced. Preferably, only flexural movements are then admitted, a fact which is of advantage for certain applications.

In a further embodiment shown in Fig. 18., rod-shaped element 503 has rigid sections 507, 508 and elastic section 590 as in the preceding embodiment. In coaxial bore hole 510 is provided a tensile element 512, such as a wire, that is attached to rigid sections 507,508 by fixing elements, such as clamping screws 513, under tension. In operation, this makes it possible to pre-stress elastic section 590.

The features of the described embodiments can be combined with each other. For example, rod-shaped element 501 can also have a core and/or shaped sections for obtaining oriented rigidity. In one variant of embodiment 502, the elastic section is waisted uniformly at one position or several concave areas shaped at uniform distances are provided in the circumferential direction in order to obtain a certain rigidity in defined directions.

In a further embodiment, the rod has several rigid sections for several elastic sections each lying between them, such that a majority of pedicle screws can be connected to each other partly rigidly, partly elastically.

In a further embodiment, a coating or a protective sleeve of biocompatible material is provided around the elastic section in order that no tissue or blood vessels or any other body material can come between the threads and as a result be injured or the function of the rod-shaped element be impaired.

In a further embodiment, monoaxial screws are provided instead of polyaxial screws or a combination of a polyaxial screw and a monoaxial screw for the stabilization device or combinations of several of these screws are used. Also, the use of hooks instead of bone screws is conceivable. In a further embodiment, the rigid sections and/or the elastic section are curved.

Figures 19 to 24 show preferred applications of the stabilization device of the invention with the rod-shaped element. In the stabilization device of Figs. 19 to 22, the rod-shaped element 502 is used which has core 511. The stabilization device is used, for example, when a slightly or moderately defective intervertebral disc 506 is to be supported and the action of harmful forces on the intervertebral disc is to be avoided by limiting the motion of the vertebrae. Rod-shaped element 502 is rigid in the axial direction and permits neither com- pression nor extension in the axial direction. Flexural movements at an angle a to the rod axis, which for example can be up to 8 are possible, however.

Fig- 23 shows the application of the stabilization device with the rod-shaped element in the case of fusion of two vertebrae 404,45 by means of rigid element 450, for example a titanium cylinder, after removal of the natural intervertebral disc. Here, greater rigidity of the rod is desired in order that adequate movement limitation may be obtained. The slight possibility of movement of the vertebrae towards each other is, however, of advantage compared with an exclusively rigid connection, because the increased cyclical partial load stimulates bone growth and ossification thus proceeds faster.

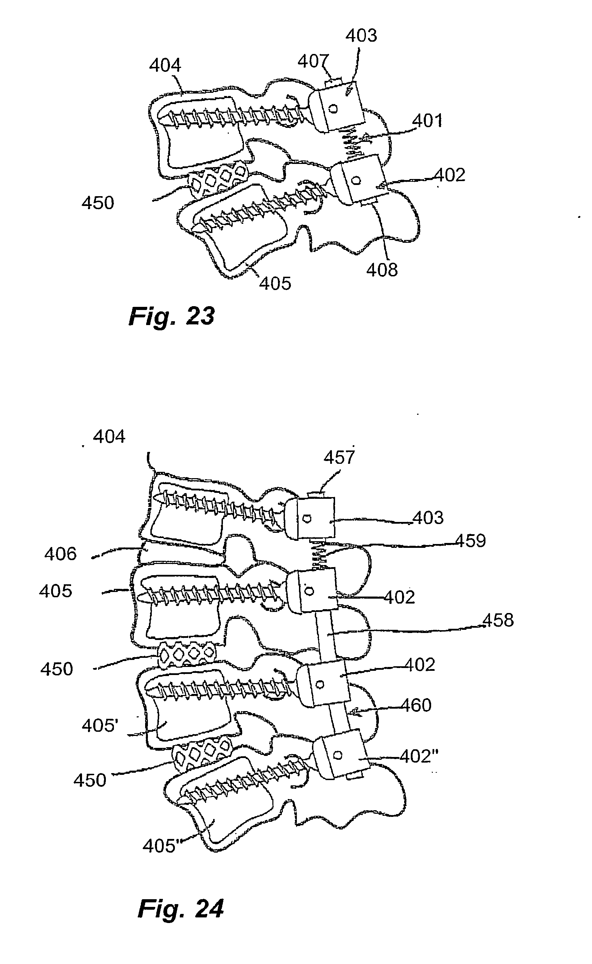

Fig. 24 shows the application of the dynamic stabilization device as the flexible end of an extended fusion in which several, in the example shown, three, vertebrae 405,405', 405" are fused to each other by rigid elements 450 and posteriorly connected via a rigid rod 460. The natural intervertebral disc 406 contacting the last vertebra 405 of the fused chain, and the next vertebra 4, are subject to disproportional loading which leads to higher wear of intervertebral disc 406. To protect this neighboring segment against unusual movement and thus increased loading, the stabilization device is provided as movement limitation. Rod 460 has in this sample embodiment a rigid section 458 which is dimensioned such that three pedicle screws 402,402', 402"can be connected to it. Elastic section 459 is provided contiguous to this, and at the end, again, a rigid section 457 for connecting to pedicle screw 403.

In the manufacturing methods of the invention for the rod-shaped element, a rigid rod of a desired diameter is provided from a body-compatible material, such as titanium. Then, in a section between the ends of the rods, elastic section 409 is made in the form of a helical spring by means of milling. Core 410 penetrating through the spring section is then, if desired, bored, as a result of which rod 401 is produced.

To generate rod 502, core 511 is either left or a separate core is subsequently pushed in.

To generate rod 500, a rod having a diameter that meets the diameter of the desired elastic section 590 is provided as starting material. The helical spring is then generated, e. g. by milling. Then, rigid end sections 507,508 are turned to the desired diameter.

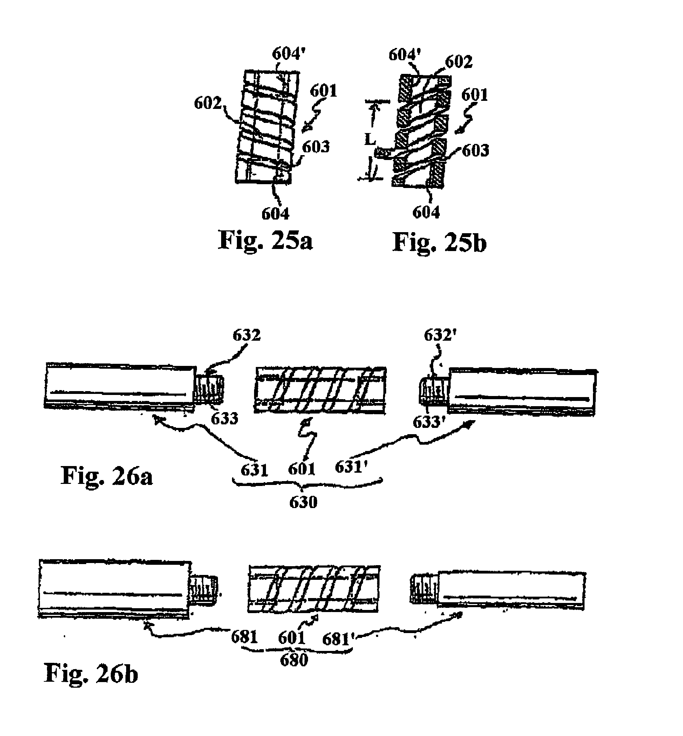

To produce rod 501, material is removed in one area, at places of the elastic section that are offset from each other by 180 around the circumference, in order to thereby generate an oriented waist.
The flexible element 601 shown in figures 25a and 25b consists of a cylindrical tube with a continuous coaxial bore hole 602 and a recess 603 extending in the wall for a predefined length in the form of a helix with a predefined pitch along the direction of the cylinder axis, which ends in hole 602 in radial direction. Thereby, a helical spring is formed. The length of the helix-shaped recess in the direction of the cylinder axis, the height of the recess, the pitch of the helix, and the diameter of the coaxial bore hole are selected such that a desired stiffness of the helical spring with respect to axial forces, bending forces, and torsional forces acting an the spring element is provided. Adjacent to each of its free ends, flexible element 601 comprises internal threads 604,604'that extend along a predetermined length.

The external diameter of the flexible element is selected according to the corresponding application.

In a first application example, shown in Fig. 26, spring element 601 is an integral part of an elastic rod-shaped element 630. The elastic rod-shaped element 630 consists of flexible element 601 and two cylindrical rod sections 631,631'each comprising at their end a cylindrical projection 632,632'with an external thread 633,633'that acts in conjunction with internal thread 604,604' of flexible element 601. In this embodiment, the rod sections and the flexible element have essentially identical external diameters. The length of rod sections 631, 631' and of spring element 601 can be selected independently of each other with respect to a desired application. For example, the rod-shaped element is used to connect pedicle screws at the spinal column. Owing to the elastic properties of spring element 601, the rod-shaped element 630 thus formed absorbs compression, extension, bending and torsional forces to a predetermined degree.

Fig. 26b shows an elastic rod-shaped element 680 that differs from elastic rod-shaped element 630 in that a first rigid rod section 681 has a larger external diameter than flexible element 601, and the second rigid rod section 681' has a smaller external diameter than flexible element 601. As an alternative, it is also possible for both rod sections to have a larger or smaller diameter than the spring element

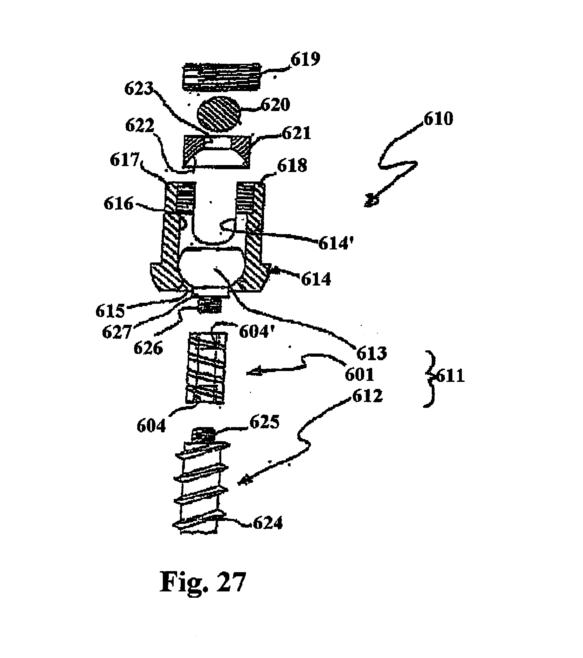

Fig. 27 shows a second application example of the flexible element 601. Here, flexible element 601 is an integral part of a bone anchoring element 610 that is provided in the form of a polyaxial bone screw. The polyaxial bone screw comprises a screw element 611, which consists of the flexible element 601, a shaft 612 with a tip that is not shown here und a screw head 613.

The shaft 612 comprises a bone thread 624 for screwing into the bone and a cylindrical projection 625 with an external thread that acts in conjunction with internal thread 604 of flexible element 601.

Screw head 613 comprises a cylindrical projection 627 and, adjacent to that, similar to shaft 612, a cylinder-shaped projection 626 with an external thread that acts in conjunction with internal thread 604' of flexible element 601.

Screw element 611 is held in a receiving part 614 such that it can be swiveled in its no-load state. Receiving part 614 is essentially cylindrical in shape and, at one of its ends, comprises a first bore hole 615 that is aligned in an axially symmetric direction and has a diameter that is larger than that of shaft 612 and smaller than that of screw head 613. Furthermore, receiving part 614 comprises a coaxial second bore hole 616 that is open at the end opposite to the first bore hole 615 and whose diameter is dimensioned such that the screw element can be passed through the open end and, with its shaft, through first bore hole 615 until screw head 613 abuts against the edge of first bore hole 615. Receiving part 614 comprises a T-shaped recess 614' that extends from the free end in the direction of first bore hole 615 and forms two free legs 617,618. In a region adjacent to their free end, legs 617,618 8 comprise an internal thread that acts in conjunction with a corresponding external thread of an internal screw 619 for fixing a rod 620.

Furthermore, a pressure element 621 for fixing screw head 613 is provided in receiving part 614 with pressure element 621 being provided such that it comprises a spherical recess 622 on its side facing screw head 613 with spherical recess 622 having a radius that is essentially identical to the radius of the spherical segment-shaped section of screw head 613. The external diameter of pressure element 621 is selected such that pressure element 621 can be displaced within receiving part 614 in the direction of screw head 613.

Furthermore, pressure element 621 comprises a coaxial bore hole 623 for access to a recess (not shown) in screw head 613 for engagement by a screwing-in tool.
In operation, shaft 612 is screwed into internal thread 604 of flexible element 601 with its cylindrical projection 625 and screw head 613 is screwed into internal thread 604' with its cylindrical projection 626 so as to form a screw element 611. With shaft 612 leading, screw element 611 thus formed is then introduced through the second orifice into receiving part 614 until screw head 613 abuts against the edge of first bore hole 615. Thereafter, pressure element 621 is introduced through second bore hole 616 and into receiving part 614 with the spherical recess leading. Then screw element 611 is screwed into the bone or vertebra.

Finally, rod 620 is placed in receiving part 614 between the two legs 617 and 618 the angular position of the receiving part relative to the screw element is adjusted and fixed with internal screw 619. The elastic section permits a limited degree of motion about the resting position.

The polyaxial screw is not limited to the embodiment described above, but rather can be any other polyaxial screw with a three-piece screw element according to the description above.

Accordingly, first bore hole 615 of the embodiment shown in Fig. 27 can have a smaller diameter than shaft 612, if, in operation, screw head 613, with its cylindrical projection 626 leading, is introduced through second bore hole 616 into receiving part 614 first, before flexible element 601 and shaft 612 are screwed onto screw head 613. In this case, it is sufficient for the first bore hole 615 to have a larger diameter than cylindrical projection 626 and cylindrical section 627. Alternatively, screw head 613 can also be provided without cylindrical section 627. In this case, the bore hole must only be large enough to allow projection 26 to be guided through.

However, the receiving part can also be provided such that the screw element can be inserted from below and is clamped in the receiving part by means of a pressure element. In this case, the bore hole 615 shown in Fig. 27 is larger than the diameter of screw head 613.

The rod fixation is not limited to the internal screw shown in Fig. 27, but an additional external nut can be provided or any known type of rod fixation can be used.

If flexible element 601 projects beyond the surface of the bone at least in part, flexible element 601 is capable of absorbing bending forces as well as tension and pressure forces.

When the spring element no longer projects beyond the surface of the bone, screw element 611 is still capable of giving way in response to a movement of the bone or vertebra. This prevents the development of unfavourable tension.

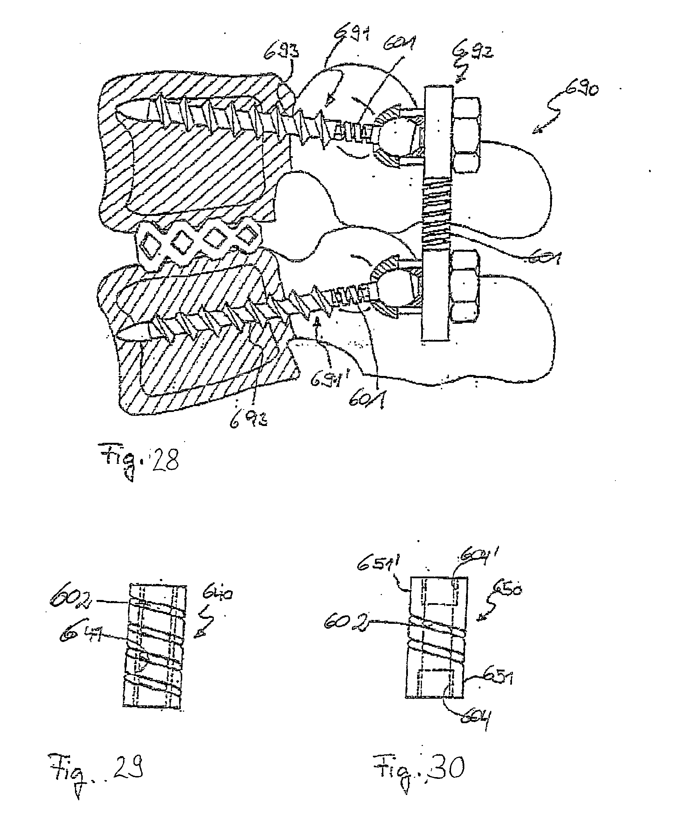

Fig. 28 shows a stabilization device 690 for the spinal column, wherein two bone anchoring elements 691,691'with screw elements 693 and an elastic rod-shaped element 692 each provided with a flexible element 601 according to the invention, are used for connecting the two bone anchoring elements. The multiple-piece design of the elastic rod-shaped element and the screw element permits to obtain stabilization devices 690 with a wide variety of features by combining only a few basic elements. The stabilization device does not necessarily have to comprise bone anchoring elements with a flexible element and a rod-shaped element provided with the flexible element. Depending on the field of application, it is also possible to provide only a rod-shaped element with flexible element and bone anchoring elements with rigid screw elements.

Fig. 29 shows a flexible element 640. Flexible element 640 differs from flexible element 601 only in that an internal thread 641 that extends along the entire length of the flexible element is provided instead of the two internal threads 604,604'.

Fig. 30 shows a flexible element 650. In contrast to the preceding embodiments it comprises rigid end sections 651 and 651'and a reduced number of helical turns as compared to the preceding embodiments. This permits to design the elasticity of the spring element independent of the length of the spring element.

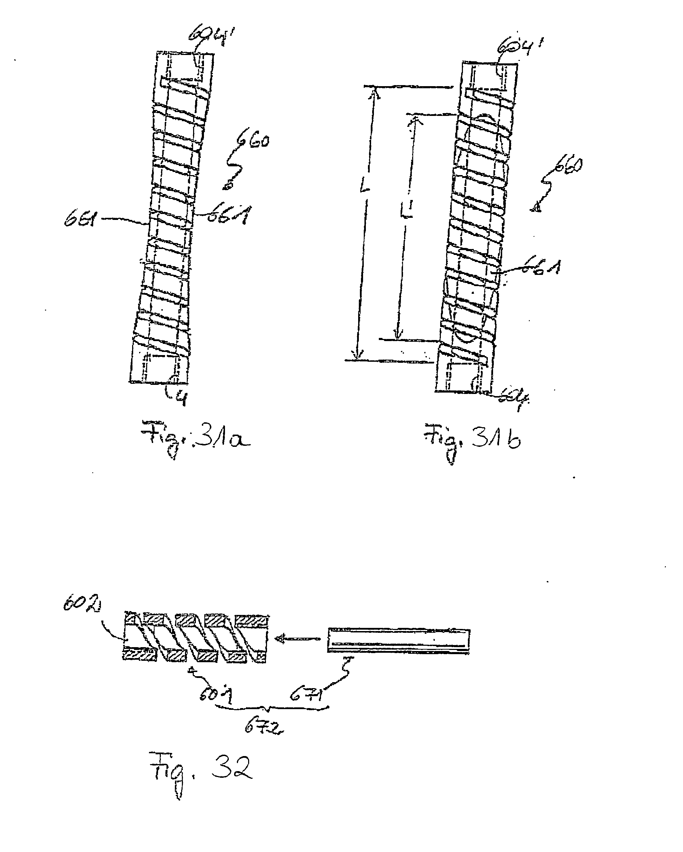

Figures 31a and 31b show a spring element 660 which, in contrast to the preceding embodiments, comprises two regions 661 that are offset by 180 degrees relative to each other and are concave in shape towards the center axis. The length L' of regions 661 in the direction of the center axis is no more than equal to the length L of the helix, and the radius of curvature of the shaped regions 661 is such that the turns of the helical spring are not interrupted. Owing to this design, the spring element has a waisted shape in a direction that is perpendicular to the center axis, thus possessing less stiffness in this direction. This provides for the flexible element to have oriented stiffness which suits the purpose of certain applications.

Fig. 32 shows a flexible element 672 that comprises a rod-shaped core 671 that is slid into the hole. On the other hand, the core can serve as a limit stop in case flexible element 672 is subjected to pressure forces. On the other hand, core 671 can be used to increase the stiffness of flexible element 672 with respect to bending forces.

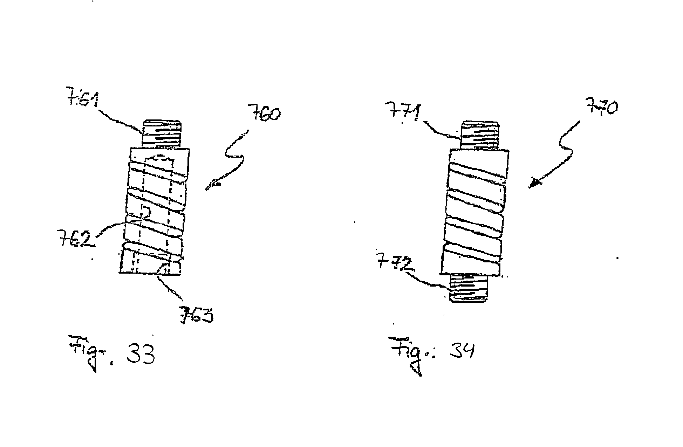

A spring element 760 shown in Fig. 33 comprises on its one end a cylindrical projection 761 with an external thread instead of a bore hole with an internal thread as in the preceding embodiment. Accordingly, the element to be connected to this end of the spring element is provided with a bore hole with a corresponding internal thread. The other end of the flexible element is provided with a pocket bore hole 762 in which an internal thread 763 is provided adjacent to the end of the spring element like in the embodiment described above.

A spring element 770 shown in Fig. 34 comprises on each of its ends a cylindrical projection 771,772 with an external thread.

In a modification of the preceding embodiments, the flexible element does not comprise a continuous bore hole.

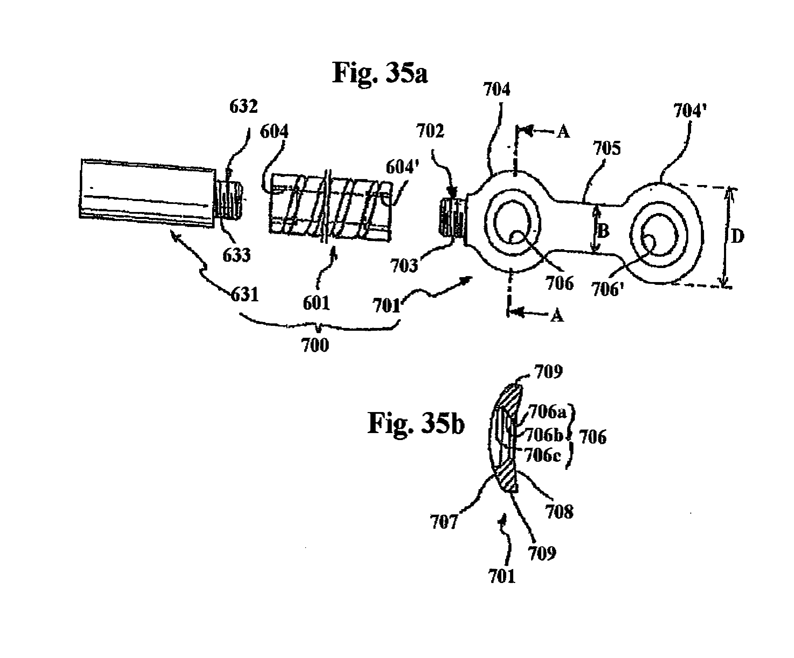

As a further application example of flexible element 601 according to the invention Fig. 35a shows the exploded view of a connection element 700 that consists of a rod-shaped element 631, a flexible element 601 and a plate 701. Rod-shaped element 631 comprises a cylindrical projection 632 with an external thread 633 for screwing into the internal thread 604 that is adjacent to the one end of flexible element 601. Plate 701 also comprises a cylindrical projection 702 with an external thread 703 for screwing into the internal thread 604'that is adjacent to the other end of flexible element 601. The plate consists of two sections 704, 704' that are circular in the top view and connected to each other by means of fin 705. The width B of fin 705 is smaller than the diameter D of circular sections 704,704'. Two bore holes 706, 706' through the plate for countersunk screws are provided coaxial to the circular sections. As shown in Fig. 35b, the first side 707 of the plate has a convex curvature, whereas the second side 708 of the plate has a concave curvature for abutment of this side against a bone. The different radii of curvature of the two sides 707,708 of plate 701 cause plate 701 to taper towards the lateral edges 709. This allows the plate to be both stable and space-saving. As shown in Fig. 35b, bore holes 706,706' comprise, adjacent to the second side 708 an orifice 706a and, adjacent to the orifice, a cone-shaped first section 706b and a second section 706c that is adjacent to the first section and first side 707. Their shape makes these bore holes 706,706'suitablc for receiving countersunk screws. The shape of bore holes 706,706' can also be different from the shape described above as long as they are suitable to receive a countersunk screw.

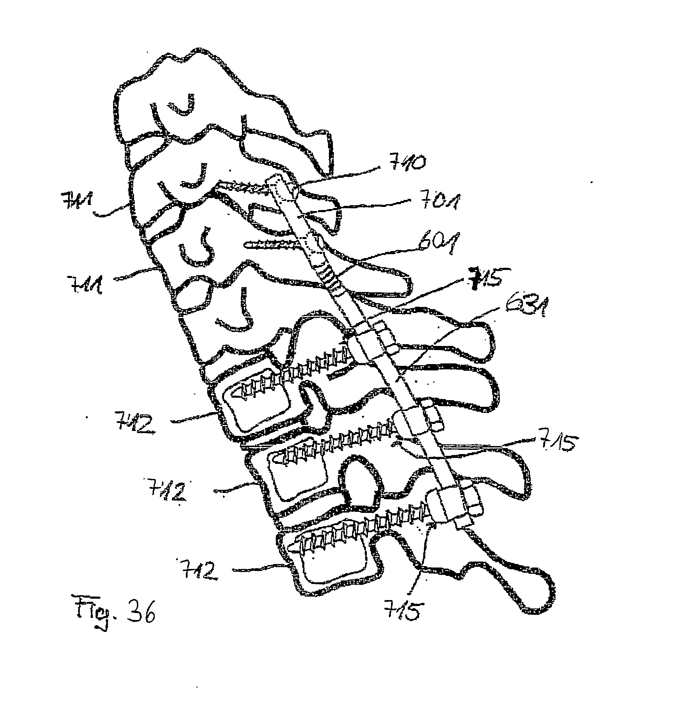

Fig. 36 shows an application example of the connection element 700 of Fig. 35a, in which plate 701 is fixed from the posterior side to two vertebrae 711 of the cervical spine by means of two bone screws 710 and in which the rod-shaped element 631 that is connected to the plate by means of a flexible element 601 is anchored in vertebrae 712 of the thoracic spine by means of three bone anchoring elements 715.

An application example not within the scope of the claims, in which the spring element 601 according to the invention is used in a dynamic pelvis stabilization device 730 is shown in Fig. 37a. Such a dynamic pelvis stabilization device may consist of bone anchoring elements 728,728', 728"that are connected to each other by means of rod-shaped elements 631,631', 631" and flexible elements 601,601'.

Like the two other bone anchoring elements 728', 728" the bone anchoring element 728 consists of two halves 725,731 that are screwed to each other by means of a screw 727 engaging a thread 734 in the first half 725 and a thread 735 in the second half 731. The top view shown in Fig. 37a only shows the top half 725. Rod-shaped element 631 is clamped between the two halves 725,731 mentioned previously in a recess 732 in the first half 725 and in a recess 733-in the second half 731 such that bone anchoring element 728 is firmly connected to rod-shaped element 631. Moreover, both halves 725,731 are each provided with a bore hole 736 or 737, which are in a coaxial alignment in the assembled state.

Adjacent to bore hole 736, a spherical recess 738 and adjacent to bore hole 737, a spherical recess 739 is provided which serve to receive a bone screw 726. Bone screw 726 comprises a shaft-shaped section 751 with an external thread 752 for screwing into the bone, and a spherical segmentshaped head section 753 with a radius that is essentially identical to the radius of spherical recesses 738,739.

Like the bone anchoring element connection element 724 consists of two halves 722 of which only one is depicted in the top view shown in Fig. 37a. Guided within a recess, rod-shaped element 631 is clamped between these two halves 722 mentioned previously such that connection element 724 is firmly connected to rod-shaped element 631.

Rod element 721 consists of a ball-shaped head section 72 1 b and a shaft section 721a. Head section 721b is clamped between the two halves 722 in a recess not shown here and thus is connected to the two halves 722 such that it can be fixed in a certain pivot position. At its end opposite to head section 721b, shaft section 721a comprises a cylindrical projection (not shown) with an external thread that is screwed into the internal thread (not shown) of flexible element 601'.

The manufacture of a flexible element by means of filling can start with a cylinder made of a biocompatible material, e. g. titanium, with a predetermined external diameter, in which a recess is then milled with a thin disk milling cutter along a helix whose main axis is collinear to the main axis of the cylinder. Subsequently, a bore hole is formed along the main axis of the cylinder over the entire length of the cylinder such that helix-shaped recess ends in bore hole. For the stability of flexible element, the runout of the helix at the transition between the helical section and the end-side section of the spring element is of major significance. It is therefore necessary to finish the runout of the helix at both ends of the helix with a end-milling cutter such that the sharp edge on the inside of the bore hole is removed. For this purpose, the runout is milled with an end-milling cutter at an angle tangential to the contour of the helix. Subsequently, the component is deburred on its inside and outside. Finally, an internal thread is formed in each of the two end sections of the bore hole.

As an alternative to milling, the flexible element 800 is manufactured from the cylindrical body by wire-cut-EDM, laser treatment or water jet treatment. As is shown in Fig. 38, this also starts with a cylinder with a predetermined external diameter D', in which is formed a bore hole 801 along the main axis A over the entire length of the cylindrical body in the subsequent step. Then a cut is made in the wall of the hollow cylinder thus formed along a helix 802 using one of the procedures mentioned above depending an the thickness of the wall. The runout 803 of helix 802 is formed to take the shape of a quarter circle such that the finishing of runout 803 in an additional work step as compared to the milling procedure can be dispensed with. Moreover, it is not necessary to debur in this manufacturing procedure. The shape of the runout does not necessarily have to be a quarter circle, but rather can be any other shape, such as the shape of another section of a circle by which the load peaks in the material can be kept low during operation.

And finally, an internal thread is formed in each of the two end sections of bore hole like in the manufacturing procedure using milling.

In a modification, the procedures described above arc modified by replacing at least one of the internal threads by turning on a lathe a cylindrical projection with an external thread at the start of the procedure. In this case, the diameter of the bore hole must be smaller than the diameter of the cylindrical projection.

In a further modification of the manufacturing procedure, the spring element is manufactured without a continuous bore hole.

## Claims

1. Implant for temporary or permanent introduction into a human or animal body comprising a system of a space holder (10) for vertebrae and/or intervertebral dises and pedicle screw arrangements with a connection rod (20), the space holder (10) and a pedicle screw arrangement each comprising at least one biocompatible material with a shape that is oriented to fulfill one or more first functions and at least one or more areas (1, 18) in which, as second function, elasticity or mobility is provided, with the implant having material recesses (7, 19) in the area or areas which serve to locally reduce rigidity and are provided in addition to the shape caused by the first functions.

2. Implant of claim 1,
**characterized by the fact that**
the implant or at least parts thereof with areas of first and second functions are formed integrally from one material.

3. Implant of any of the previous claims,
**characterized by the fact that**
the area or areas are formed with material recesses as compression or expansion zones, torsion zones and/or as articulated joints, which are especially integrally connected with other functional areas.

4. Implant of any of the previous claims,
**characterized by the fact that**
the biocompatible material is of a rigid, especially under the intended conditions of use, flexuraly rigid material.

5. Implant of any of the previous claims,
**characterized by the fact that**
the biocompatible material is selected from the group that comprises titanium and alloys thereof as well as plastics.

6. Implant of any of the previous claims,
**characterized by the fact that**
the material recess (7, 19) is formed as a groove-like recess and/or as an open aperture of the wall, especially in a helical shape.

7. Implant of any of the previous claims,
**characterized by the fact that**
two material recesses are formed as a groove-like recess and/or as an open aperture arranged twin-track helically inside each other.

8. Implant of any of the previous claims,
**characterized by the fact**
that the implant comprises an implant part of a flexible material, especially of an elastomer, that acts together with the implant part with material recesses to achieve a flexibility such that a definitive rigidity or mobility of the overall implant can be set.

9. Implant of any of the previous claims,
**characterized by the fact that**
the space bolder (10) has space-holder and weight-transfer function as first functions and/or the connection rod (20) for pedicle screw arrangements has supporting and connection function as first functions.

10. Implam of any of the previous claims,
**characterized by the fact that**
the space holder has a tube-like body (1) and, on the ends of the tribe-like body, has means (2) for connecting to adjacent body parts or other implants or implant parts, with the material recesses in the tube-like body being provided, such that the implant is compressible and extensible in the axial direction and, with reference to the means of connection (2) provided on the ends is bendable about a radial turning axis (13) and torsionable about an axial rotating axis.

11. Implant of claim 10,
**characterized by the fact that**
the tube-like body (1) is surrounded by a sleeve consisting of an elastic biocompatible material or/and is provided with a core consisting of an elastic biocompatible material.

12. Implant of claim 11,
**characterized by the fact that**
the sleeve and/or the core are held by end plates arranged on the tube-like body integrally and/or detachably, especially by a screw or thread connection.

13. Implant of any of claims 11 or 12,
**characterized by the fact that**
the elastic material is an elastomer.

14. Implant of any of the previous claims,
**characterized by the fact that**
the implant and especially the tube-like body, expressed in terms of its longitudinal direction, is elastically extensible or compressible by 0.5 to 20%, especially 1 to 15%.

15. Implant of any of the previous claims,
**characterized by the fact that**
the implant and especially the tube-like body (1) is elastically bendable about a radial axis (3), such that the means of connection (2) provided at the ends can pivot by approximately 0.5 to 10°, especially 1 to 6° from the longitudinal axis (12) of the tube-like body.

16. Implant of any of the previous claims,
**characterized by the fact that**
the implant and especially the tube-like body is torsionable about the axial axis by 0.5 to 10°, especially 1 to 6°.

## Patentansprüche

1. Implantat für den zeitweiligen oder permanenten Einsatz in einem menschlichen oder tierischen Körper, das ein System mit einem Platzhalter (10) für Wirbel und/oder Bandscheiben und Pedikelschraubenanordnungen mit einem Verbindungsstab (20) umfasst, wobei der Platzhalter (10) und eine Pedikelschraubenanordnung jeweils mindestens ein biokompatibles Material umfassen mit einer Form, die darauf ausgerichtet ist, einer oder mehreren ersten Funktionen zu genügen, und mit mindestens einen oder mehr Bereichen (1, 18), in welchen als zweite Funktion Elastizität oder Beweglichkeit bereitgestellt wird, wobei das Implantat Materialausnehmungen (7, 19) in dem Bereich oder Bereichen aufweist, die dazu dienen, lokal die Steifheit zu reduzieren und die zusätzlich zu der durch die ersten Funktionen bedingten Form bereitgestellt werden.

2. Implantat nach Anspruch 1,
**dadurch gekennzeichnet, dass**
das Implantat oder wenigstens Teile davon mit Bereichen mit ersten und zweiten Funktionen wird integral aus einem Material geformt.

3. Implantat nach einem der vorhergehenden Ansprüche,
**dadurch gekennzeichnet, dass**
der Bereich oder die Bereiche mit Materialausnehmungen als Kompressions-oder Expansionszonen, Torsionszonen und/oder Gelenkverbindungen ausgebildet sind, die insbesondere integral mit anderen funktionalen Bereichen verbunden sind.

4. Implantat nach einem der vorhergehenden Ansprüche,
**dadurch gekennzeichnet, dass**
das biokompatible Material aus einem steifen, insbesondere bei den beabsichtigten Nutzungsbedingungen biegesteifen Material ist.

5. Implantat nach einem der vorhergehenden Anspruche,
**dadurch gekennzeichnet, dass**
das biokompatible Material aus der Gruppe ausgewählt ist, die Titan und dessen Legierungen sowie Kunststoff umfasst.

6. Implantat nach einem der vorhergehenden Ansprüche,
**dadurch gekennzeichnet, dass**
die Materialausnehmung (7, 19) als ein nutenartige Ausnehmung und/oder als ein offener Durchbruch der Wand, insbesondere in einer schraubenförmigen Form, ausgebildet ist.

7. Implantat nach einem der vorhergehenden Ansprüche,
**dadurch gekennzeichnet, dass**
zwei Materialausnehmungen (7, 19) als nutenartige Ausnehmungen und/oder als offene Durchbrüche der Wand ausgebildet sind, die doppelspurig schraubenförmig ineinander angeordnet sind.

8. Implantat nach einem der vorhergehenden Ansprüche,
**dadurch gekennzeichnet, dass**
das Implantat einen Implantatbereich aus einem flexiblen Material, insbesondere einem Elastomer, umfasst, der zusammen mit dem Implantatbereich mit Materialausnehmungen zusammen wirkt, um eine Flexibilität zu erzielen, so dass eine definierte Steifheit oder Beweglichkeit des gesamten Implantats festgelegt werden kann.

9. Implantat nach einem der vorhergehenden Ansprüche,
**dadurch gekennzeichnet, dass**
der Platzhalter (10) eine Platzhalter - und eine Gewichtsübertragungsfunktion als erste Funktionen und/oder der Verbindungsstab (20) für die Pedikelschraubenanordnungen Stütz- und Verbindungsfunktion als erste Funktionen aufweist.

10. Implantat nach einem der vorhergehenden Ansprüche,
**dadurch gekennzeichnet, dass**
der Platzhalter einen röhrenartigen Körper (1) und an den Enden des röhrenartigen Körpers Mittel (2) zum Verbinden mit benachbarten Körperbereichen oder anderen Implantaten oder Implantatbereichen aufweist, wobei die Materialausnehmungen in dem röhrenartigen Körper bereitgestellt sind, so dass das Implantat in axialer Richtung komprimierbar und dehnbar ist und in Bezug auf die Mittel zur Verbindung (2) die an den Enden bereitgestellt sind, um eine radiale Kippachse (13) biegbar ist und um eine axiale Drehachse verdrehbar ist

11. Implantat nach Anspruch 10,
**dadurch gekennzeichnet, dass**
der röhrenartige Körper (1) von einer Hülse umgeben ist, die ein elastisches biokompatibles Material umfasst, oder/und mit einem Kern versehen ist, der ein elastisches, biokompatibles Material umfasst.

12. Implantat nach Anspruch 11,
**dadurch gekennzeichnet, dass**
die Hülse und/oder der Kern durch Endplatten gehalten werden, die an dem röhrenartigen Körper integral und/oder lösbar angeordnet sind, insbesondere mit einer Schrauben- oder Gewindeverbindung.

13. Implantat nach Anspruch 11 oder 12,
**dadurch gekennzeichnet, dass**
das elastische Material ein Elastomer ist.

14. Implantat nach einem der vorhergehenden Ansprüche,
**dadurch gekennzeichnet, dass**
das Implantat und insbesondere der röhrenartige Körper in Bezug auf die longitudinale Richtung um 0,5 bis 20 %, insbesondere 1 bis 15 % elastisch dehnbar oder komprimierbar ist.

15. Implantat nach einem der vorhergehenden Ansprüche,
**dadurch gekennzeichnet, dass**
das Implantat und insbesondere der röhrenartige Körper (1) elastisch um eine radiale Achse (3) biegbar ist, so dass die Mittel zur Verbindung (2), die an den Enden bereitgestellt werden, um ungefähr 0,5 bis 10°, insbesondere 1 bis 6° in Bezug auf die longitudinale Achse (12) des röhrenartigen Körpers gekippt werden können.

16. Implantat nach einem der vorhergehenden Ansprüche,
**dadurch gekennzeichnet, dass**
das Implantat und insbesondere der röhrenartige Körper um 0,5 bis 10°, insbesondere 1 bis 6° um die axiale Achse verdrehbar ist.

## Revendications

1. Implant pour introduction temporaire ou permanente dans le corps d'un être humain ou d'un animal comprenant un système de mainteneur d'espace (10) pour des vertèbres et/ou des disques intervertébraux et des arrangements de vis pédiculaire avec une tige de connexion (20), le mainteneur d'espace (10) et un arrangement de vis pédiculaire comprenant chacun au moins un matériau biocompatible avec une forme qui est orientée pour remplir une ou plusieurs premières fonctions et au moins une ou plusieurs zones (1, 18) dans lesquelles, comme seconde fonction, l'élasticité ou la mobilité est fournie, avec l'implant ayant des évidements de matériau (7, 19) dans la zone ou les zones qui servent à réduire localement la rigidité et qui sont de plus prévues en plus de la forme causée par les premières fonctions.

2. Implant selon la revendication 1,
**caractérisé par le fait**
**que** l'implant ou au moins des parties de celui-ci avec des zones de premières et de secondes fonctions sont formées intégralement dans un matériau.

3. Implant selon l'une quelconque des revendications précédentes,
**caractérisé par le fait que**
la zone ou les zones sont formées avec des évidements de matériau comme des zones de compression ou d'expansion, des zones de torsion et/ou des joints articulés qui sont en particulier intégralement reliées à d'autres zones fonctionnelles.

4. Implant selon l'une quelconque des revendications précédentes,
**caractérisé par le fait que**
le matériau biocompatible est un matériau rigide, en particulier dans les conditions d'utilisation, un matériau rigide quant à la flexion.

5. Implant selon l'une quelconque des revendications précédentes,
**caractérisé par le fait que**
le matériau biocompatible est sélectionné dans le groupe qui comprend le titane et les alliages de celui-ci ainsi que les plastiques.

6. Implant selon l'une quelconque des revendications précédentes,
**caractérisé par le fait que**
l'évidement de matériau (7, 19) est formé comme un évidement comme une rainure et/ou comme un orifice ouvert de la paroi, en particulier de forme hélicoïdale.

7. Implant selon l'une quelconque des revendications précédentes,
**caractérisé par le fait que**
deux évidements de matériau sont formés comme un évidement de type rainure et/ou comme un orifice ouvert arrangé hélicoïdal double voie à l'intérieur l'un de l'autre.

8. Implant selon l'une quelconque des revendications précédentes,
**caractérisé par le fait que**
l'implant comprend une partie d'implant en matériau flexible, en particulier en un élastomère, qui agit ensemble avec la partie d'implant avec des évidements de matériau pour obtenir une flexibilité telle qu'une rigidité ou mobilité définitive de l'ensemble de l'implant peut être réglée.

9. Implant selon l'une quelconque des revendications précédentes,
**caractérisé par le fait que**
le mainteneur d'espace (10) a une fonction de mainteneur d'espace et de transfert de poids comme premières fonctions et/ou la tige de connexion (20) pour les arrangements de vis pédiculaire a une fonction de support et de connexion comme premières fonctions.

10. Implant selon l'une quelconque des revendications précédentes,
**caractérisé par le fait que**
le mainteneur d'espace a un corps tubulaire (1) et a, sur les extrémités du corps tubulaire, des moyens (2) pour la connexion à des parties de corps adjacentes ou à d'autres implants ou parties d'implant, les évidements de matériau étant prévus dans le corps tubulaire de telle manière que l'implant est compressible et extensible dans le sens axial et, en se référant aux moyens de connexion (2) prévus aux extrémités, est pliable autour d'un axe de rotation radial (13) et est capable de torsion autour d'un axe de rotation axial.

11. Implant selon la revendication 10,
**caractérisé par le fait**
**que** le corps tubulaire (1) est entouré par un manchon consistant en un matériau biocompatible élastique ou/est pourvu d'un noyau consistant en un matériau biocompatible élastique.

12. Implant selon la revendication 11,
**caractérisé par le fait**
**que** le manchon et/ou le noyau sont maintenus par des plaques d'extrémité arrangées sur le corps tubulaire en une partie intégrale et/ou de manière détachable, en particulier par une jonction à vis ou à filet.

13. Implant selon l'une quelconque des revendications 11 ou 12,
**caractérisé par le fait**
**que** le matériau élastique est un élastomère.

14. Implant selon l'une quelconque des revendications précédentes,
**caractérisé par le fait que**
l'implant, et en particulier le corps tubulaire, est extensible ou compressible élastiquement, ceci étant exprimé pour ce qui est de son sens longitudinal, de 0,5 à 20 %, en particulier de 1 à 15 %.

15. Implant selon l'une quelconque des revendications précédentes,
**caractérisé par le fait que**
l'implant, et en particulier le corps tubulaire, est pliable élastiquement autour d'un axe radial (3) de telle manière que les moyens de connexion (2) prévus aux extrémités peuvent pivoter d'environ 0,5 à 10°, particulièrement de 1 à 6° à partir de l'axe longitudinal (12) du corps tubulaire.

16. Implant selon l'une quelconque des revendications précédentes,
**caractérisé par le fait que**
l'implant, et en particulier le corps tubulaire, peut être tordu autour de l'axe axial de 0,5 à 10°, en particulier de 1 à 6°.
